# EUROPEAN PATENT SPECIFICATION

(11) **EP 3 445 336 B1**
(45) Date of publication and mention of the grant of the patent: **28.10.2020**
(21) Application number: 17716257.5
(22) Date of filing: 12.04.2017
(51) Int. Cl.: A61K 9/14, A61K 9/16, A61K 9/28, A61K 9/48, A61K 9/50, A61K 31/145, A61K 31/15, A61P 29/00, A61P 35/00

(54) **SHELLAC MICROCAPSULE FORMULATIONS AND COMPOSITIONS**
SCHELLACK-MIKROKAPSEL-FORMULIERUNGEN UND ZUSAMMENSETZUNGEN
FORMULATIONS DE GOMME-LAQUE MICROCAPSULES ET COMPOSITIONS

(30) Priority: 19.04.2016 EP 16165990
(43) Date of publication of application: 27.02.2019
(73) Proprietor: CONARIS Research Institute AG, 24118 Kiel (DE); Christian-Albrechts-Universität zu Kiel, 24118 Kiel (DE)
(72) Inventor: WÄTZIG, Georg, 24118 Kiel (DE); SCHWARZ, Karin, 24118 Kiel (DE); KEPPLER, Julia, 24118 Kiel (DE); THEISMANN, Eva-Maria, 24118 Kiel (DE); KNIPP, Jörg, 24118 Kiel (DE); ELLRICHMANN, Mark, 24105 Kiel (DE); SCHREIBER, Stefan, 24105 Kiel (DE)
(74) Representative: Eisenführ Speiser
(86) International application number: PCT/EP2017/058741
(87) International publication number: WO 2017/182350

(56) References cited:
- WO-A1-2010/014011
- WO-A1-2013/087391
- WO-A2-2008/091870
- US-B1- 6 620 431
- FARAG Y ET AL: "Physiochemical Properties of Various Shellac Types", INTERNET CITATION, 1 May 2005 (2005-05-01), pages 33-39, XP002689795, ISSN: 1521-298X Retrieved from the Internet: URL:http://www.dissolutiontech.com/DTresou r/200905Articles/DT200905_A04.pdf [retrieved on 2013-01-08]

## Description

### FIELD OF THE INVENTION

The present invention relates to a microcapsule, comprising a core containing an active substance, such a microcapsule for use as a medicament, medical product, diagnostic product, nutraceutical, dietary supplement, food ingredient or food, and the use of such a microcapsule for chromoendoscopy of the small and/or large intestine for the diagnosis, therapy and/or prophylaxis of diseases and/or syndromes associated with and/or accompanied by intestinal inflammation, dysplasia, carcinogenesis and/or changes in the intestinal epithelium and/or intestinal mucosa and/or intestinal wall. The present invention further relates to formulations and compositions comprising such a microcapsule and a method for producing for such a microcapsule.

### BACKGROUND

Most pharmaceutical formulations cannot be used as nutraceuticals, dietary supplements or functional food, which could be a mainstay of future personalised medicine and prevention. In nutritional formulations, all additives must be approved as a food additive or have GRAS (Generally Recognized As Safe) status. In contrast to synthetic polymers used in many pharmaceuticals, enteric coatings derived from natural components are biodegradable, relatively abundant and have no daily intake limits (Czarnocka & Alhnan 2015, Int. J. Pharm. 486:167). However, a recent comparative study concluded that "none of the GRAS-grade coatings fully complied with the different biological demands of delayed release coating systems" (Czarnocka & Alhnan 2015, Int. J. Pharm. 486:167).

Shellac coating has been described as one option in formulations aiming at systemic delivery of active substances for nutritional or medical purposes (see, e.g., PCT/US1998/015990; PCT/EP2001/003192; PCT/US2008/051662; Limmatvapirat et al. 2007, Eur. J. Pharm. Biopharm. 67:690; Farag & Leopold 2011, Eur. J. Pharm. Sci. 42:400; Czarnocka & Alhnan 2015, Int. J. Pharm. 486:167).

Shellac is the purified form of the natural resin lac, the resinous secretion of the scale insect *Kerria lacca* (Buch et al. 2009, Drug Dev. Ind. Pharm. 35:694; Chen et al. 2011, J. Insect Sci. 11:106). Shellac has good coating properties and GRAS status (Czarnocka & Alhnan 2015, Int. J. Pharm. 486:167). Moreover shellac's dissociation is pH-dependent and possesses good resistance to gastric fluid due to its acidic character (Limmatvapirat et al. 2007, Eur. J. Pharm. Biopharm. 67:690; Czarnocka & Alhnan 2015, Int. J. Pharm. 486:167). In contrast to other food-grade enteric coatings, shellac inherently has a prolonged drug release after the pH change from the acidic gastric environment to the near-neutral pH of the lower small intestine (Limmatvapirat et al. 2007, Eur. J. Pharm. Biopharm. 67:690; Czarnocka & Alhnan 2015, Int. J. Pharm. 486:167). Without further excipients or modifications, however, shellac coating with its endogenous dissolution pH of approximately 7.3 is only suitable for colonic targeting of protected substances (Farag & Leopold 2011, Eur. J. Pharm. Sci. 42:400).

A closer look at the literature on shellac-based controlled release formulations reveals different coating and subcoating strategies. Czarnocka & Alhnan (2015, Int. J. Pharm. 486:167) performed a subcoating with 10% (w/v) of the enterically inactive cellulose ether Methocel E5 (2.0% weight gain) in order to prevent interactions of the protected drug core with the enteric shellac coating. Farag & Leopold (2011, Eur. J. Pharm. Sci. 42:400) investigated different subcoatings for shellac and found, e.g., that a subcoating of citric acid (with polyvinylpyrrolidone as a carrier) delayed release at pH 6.8, because the beginning swelling and dissolution of the shellac coating allowed a partial dissociation of the underlying citric acid, leading to a pH reduction at the subcoat-coat interface and, in turn, to a reduced dissociation of the acid-stabilised shellac coating. In contrast to this study, Pearnchob et al. (2004, J. Control Release 94:313) found that the addition of organic acids (sorbic, benzoic, fumaric, adipic or citric acid) as pore formers and plasticizers in ethanolic and aqueous shellac systems rather accelerated release at pH 6.8. An important aspect are coating defects, which can be limiting for the release profiles of shellac granulates. Förmer et al. (2006, Pharmazie 61:1005) prepared pellets by powder layering of ascorbic acid on nonpareil pellets with an ethanolic shellac solution as a binder and a final coating using an ethanolic shellac solution containing 10% of tartaric acid as a plasticizer, resulting in film thickness of 2-3 mm. Purified talc powder was used as an antiadherent. Interestingly, accumulated talc particles not incorporated completely into the shellac film caused surface defects and ultimately defined the release profile of the pellets (Förmer et al. 2006, Pharmazie 61:1005). Ichikawa et al. (1991, J. Pharm. Sci. 80:1062) used sodium hydrogen carbonate (sodium bicarbonate) as a carbon dioxide source in an effervescent layer below a swellable layer which contained, among several other components, also shellac. However, in contrast to the use of organic acids as described above, the sodium bicarbonate was not used for pH modification in this approach.

In summary, there are several significant disadvantages even in a professional shellac-based coating like the PROTECT™ system of Sensient Pharmaceutical (St. Louis, MO, USA), leading to failures in comparative drug release and acid disintegration resistance testing (Czarnocka & Alhnan 2015, Int. J. Pharm. 486:167). Moreover, even advantageous subcoating strategies of the prior art like the citric acid subcoating described by Farag & Leopold (2011, Eur. J. Pharm. Sci. 42:400) only led to prolonged sustained release profiles for systemic exposure and could not be suitably tailored for targeted topical exposure of the intestinal epithelium.

Therefore, there is a large unmet need for - preferably nutritional - shellac formulations, that (1) can be fine-tuned to release the protected active substances in any section of the gastrointestinal tract from the beginning of the small intestine to the colon and/or (2) have low production costs and/or (3) have a favourable side effect profile, also for long-term administration, as a medicament, medical product, diagnostic product, nutraceutical, dietary supplement, food ingredient or food.

In the field of colon cancer detection, staining of the colonic mucosa before or during endoscopic examination (chromoendoscopy) by using various dyes (dye-based chromoendoscopy) or digital contrast and colour enhancements of standard endoscopy (dye-less chromoendoscopy) increases the contrast of the mucosal architecture (Tontini et al. 2016, World J. Gastroenterol. 22:1246). Particularly in inflammatory bowel diseases like ulcerative colitis with a significantly higher risk for colorectal cancer development, chromoendoscopy of the colon with targeted biopsies is recommended by health care authorities and the majority of gastrointestinal professional societies, as it has been shown to improve dysplasia detection (Barkin et al. 2012, Gastroenterol. Hepatol. 8:796; Trivedi & Braden 2013, Q. J. Med. 106:117; Sanduleanu et al. 2016, Gastrointest. Endosc. 83:213; Tontini et al. 2016, World J, Gastroenterol. 22:1246). However, a significantly increased adenoma detection rate with chromocolonoscopy has also been well established for routine screening colonoscopy (Pohl et al. 2011, Gut 60:485; Brown et al. 2016, Cochrane Database Syst. Rev. 4:CD006439). The current state of the art in intestinal dye-based chromoendoscopy is characterised by the empiric clinical use of a variety of dyes and staining solutions sprayed onto the intestinal mucosa using spraying catheters (Sanduleanu et al. 2016, Gastrointest. Endosc. 83:213) and by the recently started clinical development of oral methylene blue MMX® tablets by Cosmo Pharmaceuticals (Repici et al. 2012, Contemp. Clin. Trials. 33:260).

Dye-based chromoendoscopy employs three types of active substances: contrasting, absorptive and reactive (Barkin et al. 2012, Gastroenterol. Hepatol. 8:796; Trivedi & Braden 2013, Q. J. Med. 106:117; Sanduleanu et al. 2016, Gastrointest. Endosc. 83:213). Reactive dyes like congo red and phenol red are activated by local pH differences and therefore more useful in the stomach than in the colon. Absorptive dyes - the most widely used being methylene blue (usually as a 0.1% solution) - are less absorbed by dysplastic compared to healthy intestinal epithelium, thereby highlighting dysplastic lesions as heterogeneously stained or unstained areas. Further examples for absorptive dyes are Lugol's solution, toluidine blue and cresyl violet. Non-absorptive contrast dyes like the widely used indigo carmine (usually as a 0.1-0.8% solution) enhance mucosal abnormalities by pooling in grooves and crevices.

There are several drawbacks to the state of the art. Chromoendoscopy using spraying catheters is - despite its proven superiority in terms of cancer detection - far from being widely used, because many clinicians find the procedure "too hard, too messy and too lengthy" (Sanduleanu et al. 2016, Gastrointest. Endosc. 83:213). Oral administration of methylene blue MMX® tablets (Cosmo Pharmaceuticals) during bowel preparation has successfully passed phase 1 and 2 clinical trials (Repici et al. 2012, Contemp. Clin. Trials. 33:260; Danese et al. 2013, 8th Congress of ECCO, Poster Presentations: Clinical: Diagnosis and Outcome, P194; ClinicalTrials.gov: NCT01520337; NCT01520324). The main advantages claimed were better staining due to longer exposure to the dye (in this case, a dye differentially accumulating in normal and neoplastic or inflamed tissue), more uniform staining due to normal position of the intestines (during conventional chromocolonoscopy, patients are lying on one side), and a quicker and more comfortable procedure for the patients and endoscopy personnel. However, methylene blue is a pharmaceutical with several side effects, which enters the intestinal cells and leads to significant systemic exposure (Repici et al. 2012, Contemp. Clin. Trials. 33:260). Even though the well-known oxidative DNA-damaging properties of methylene blue observed in animal models were not yet observed with the methylene blue MMX tablets (Repici et al. 2015, Gastroenterology 148:S827-8), concerns remain as absorptive dyes like methylene blue can enter the cellular nucleus and behave like carcinogens (Barkin et al. 2012, Gastroenterol. Hepatol. 8:796; Trivedi & Braden 2013, Q. J. Med. 106:117).

In contrast to methylene blue, the non-absorbed food colour indigo carmine (E132) does not cause DNA damage to colonocytes (Davies et al. 2007, Gut 56:155). However, even though indigo carmine is poorly absorbed, it is partially metabolised by the intestinal microbiota, resulting in breakdown products like isatin-5-sulphonic acid and 5-sulphoanthranilic acid, which are subsequently absorbed by the body (EFSA Panel 2014, EFSA J. 12:3768). Similarly, there is little absorption of the intact food colour brilliant black BN (E151), but azo reduction of brilliant black BN in the gastrointestinal tract produces sulphonated aromatic amines which are well absorbed and reach the systemic circulation (EFSA Panel 2010, EFSA J. 8:1540). None of these substances is expected to have toxic or genotoxic effects, but the ideal non-absorbed dye should also not be metabolised to absorbed products. Patent blue V (E131) comes close to these requirements, as it shows minimal absorption and systemic availability and is mainly excreted unmetabolised in the feces (EFSA Panel 2013, EFSA J. 11:2818). According to the European Food Safety Authority (EFSA), the acceptable daily intake (ADI) of the food colours indigo carmine, brilliant black BN and patent blue V is 5 mg/kg body weight for preparations with at least 90% purity (EFSA Panel 2010, EFSA J. 8:1540; EFSA Panel 2013, EFSA J. 11:2818; EFSA Panel 2014, EFSA J. 12:3768). In the United States, only indigo carmine is approved for use in foods and drugs (FDA Color Additive Status List, December 2015). In general, food colourants would enable higher dye loads and lower systemic side effects.

The prior art comprises the use of food colours for chromoendoscopy. The patent portfolio of Cosmo Pharmaceuticals describes several embodiments of the chromoendoscopy dyes methylene blue, congo red, indigo carmine, toluidine blue or mixtures thereof embedded in their multi-matrix (MMX) technology, the most preferred being the clinical candidate methylene blue MMX (e.g., US8545811, PCT/EP2013/070060). PCT/US2015/016488 reports unsatisfactory results from the prior art regarding oral administration of indigo carmine (capsules) for chromoendoscopy and describes a method for oral administration of pharmaceutically acceptable liquid compositions of indigo carmine mixed with polyethylene glycol. PCT/EP2011/001183, PCT/AU2012/001315 and PCT/EP2013/068738 describe various compositions for bowel cleansing and chromoendoscopy staining with different dyes, including food colourants like patent blue V, brilliant black BN or indigo carmine.

However, it is difficult to deliver such dyes. Preferably, this delivery should be made in a formulation that is prepared only of food-grade chemicals and that can be fine-tuned according to both the delivered dyes and the targeted areas of the intestine. Therefore, there is a large unmet need in the field of chromoendoscopy for oral formulations of dyes for staining the small and/or large intestine which (1) contain mostly or only nutritional components, at least besides the active substance(s), and/or (2) contain one or more active substances, especially one or more dyes which are hardly or not absorbed and/or metabolised in the gastrointestinal tract and, thus, induce low or no systemic side effects. Such formulations should preferably deliver one or more active substances, especially one or more dyes to the small and/or large intestine, more preferably the small intestine, followed by a sustained release covering a large part of the colon.

When systematically investigating shellac formulations, the inventors of the present application surprisingly discovered and developed counter-intuitive coating and subcoating modifications, which specifically enabled the development of such formulations.

### SUMMARY OF THE INVENTION

The object of the present invention was to provide improved formulations and compositions for intestinal delivery of active substances, e.g., dyes for chromoendoscopy, preferably to the small intestine and/or the large intestine (colon).

In a broad sense, the present invention relates to a microcapsule, comprising a core containing an active substance, the use of such a microcapsule as a medicament, medical product, diagnostic product, nutraceutical, dietary supplement, food ingredient or food.

One preferred embodiment is the use of such a microcapsule containing one or more dyes suitable for chromoendoscopy of the small and/or large intestine for the diagnosis, therapy and/or prophylaxis of diseases and/or syndromes associated with and/or accompanied by intestinal inflammation, dysplasia, carcinogenesis and/or changes in the intestinal epithelium and/or intestinal mucosa and/or intestinal wall.

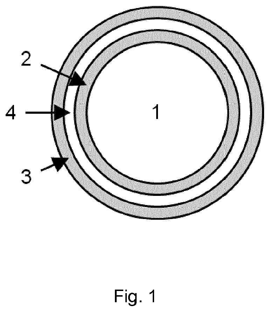

The microcapsules of the present invention (Figure 1) are characterised by a core containing an active substance (1) and a coating layer system comprising an inner layer of shellac (2), an outer layer of shellac (3) and a pH-modulating substance (4) provided between the two layers of shellac (2, 3), with the proviso that the active substance is not vitamin B3 (i.e., nicotinic acid and/or nicotinamide).

Except for vitamin B3, the active substance comprises any chemical, compound, combination, composition or mixture that has a direct effect on the gastrointestinal tract and/or an indirect effect on the body after resorption of the active substance by the gastrointestinal tract. In a preferred embodiment, the active substance comprises one or more dyes suitable for chromoendoscopy.

The pH-modulating substance comprises any chemical, compound, combination, composition, mixture or buffer system that may modulate the pH. It may be provided as an intermediate layer between the two shellac coating layers and has the function to fine-tune and control the disintegration of the shellac coatings. Depending on the pH of the active substance(s) and/or excipients in the core and the desired release profile of the microcapsule, the pH-modulating substance can be elected to be overall acidic or basic. For example, if release in the lower small intestine is desired, an overall basic pH-modulating substance may partially counteract and control the stabilising effect of an overall acidic core on the shellac coatings in order to enable release of the active substance(s) already at the pH prevalent in the lower small intestine, whereas in the case of an overall basic core, an overall acidic pH-modulating substance may subtly reduce the disintegration of the shellac coatings in order to prevent earlier release. In all microcapsules of the present invention, the novel addition of a second (inner) layer of shellac led to a surprising and counter-intuitive improvement of the performance of the formulations.

These formulations and compositions can be used for the diagnosis, prophylaxis and/or therapy of human or animal diseases, particularly for chromoendoscopy of the small and/or large intestine for the diagnosis, therapy and/or prophylaxis of diseases and/or syndromes associated with and/or accompanied by intestinal inflammation, dysplasia, carcinogenesis and/or changes in the intestinal epithelium and/or intestinal mucosa and/or intestinal wall.

According to the invention, the problem defined in the Background section is solved by a formulation or composition or diagnostic or treatment or prevention regimen as defined in the claims and described in more detail herein, which comprises controlled release of an active substance in the small intestine and/or colon. In a preferred embodiment, the active substance comprises or consists of one or more dyes suitable for chromoendoscopy like, e.g., patent blue V, indigo carmine, brilliant black BN, methylene blue, toluidine blue, cresyl violet and/or congo red. In a particularly preferred embodiment, the dye is a food colourant such as, e.g., patent blue V (E131), indigo carmine (E132) and/or Brilliant Black BN (E151).

In addition, compositions are provided which contain more than one active substance. These two or more active substances may act individually or in combination. A combination of two or more active substances may be present in the same or separate dosage forms, which may be administered simultaneously, sequentially or on separate occasions. In a preferred embodiment, the compositions are suitable for oral administration with controlled and/or delayed release of the active ingredient(s) for specific local or topical efficacy in the small intestine and/or colon. In a particularly preferred embodiment, the active substances comprise or consist of one or more dyes suitable for chromoendoscopy.

Also described are methods of diagnosing and/or treating and/or preventing one or more of the diseases and/or conditions associated with or accompanied by changes in the intestinal epithelium and/or intestinal mucosa and/or intestinal wall as described herein with a microcapsule and/or composition described herein. In addition, the invention provides the use of a microcapsule and/or composition described herein in the manufacture of a medicament and/or medical product and/or diagnostic product and/or dietary supplement for diagnosing and/or treating and/or preventing one or more of the diseases and conditions described herein.

### BRIEF DESCRIPTION OF THE DRAWINGS

Figure 1 shows a schematic representation of the microcapsules of the present invention, which are characterised by a core containing an active substance (1) and a coating layer system comprising an inner layer of shellac (2), an outer layer of shellac (3) and a pH-modulating substance (4) provided between the two layers of shellac (2, 3).

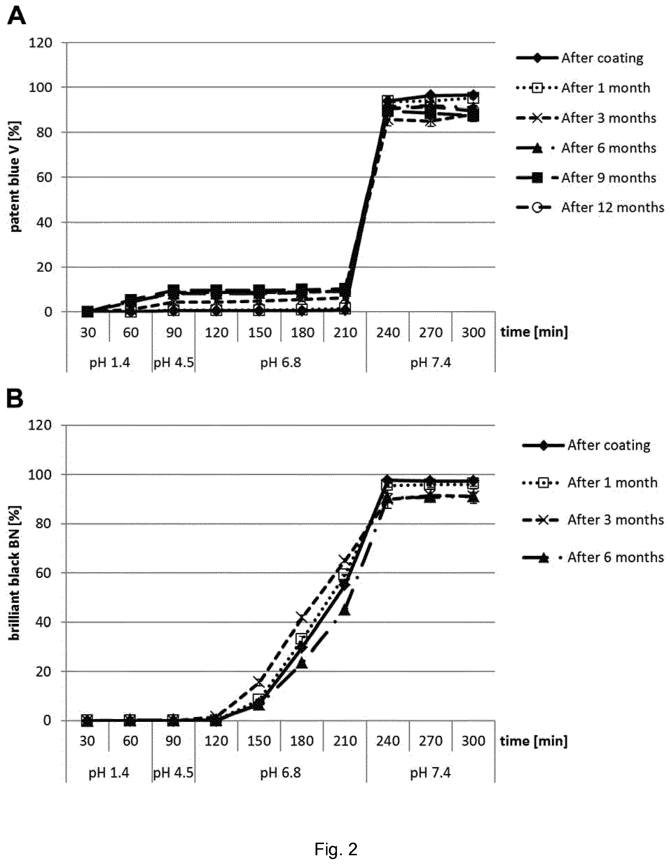

Figure 2 shows the pH-adapted *in vitro* release profiles of patent blue V (A) or brilliant black BN (B) from the microcapsule batches used for the first-in-man study immediately after coating and after up to 12 months (patent blue V) or 6 months (brilliant black BN) of storage at room temperature and protected from light. The graphs represent means ± SD (n = 2). Percentage release refers to the total mass of dye in the microcapsules.

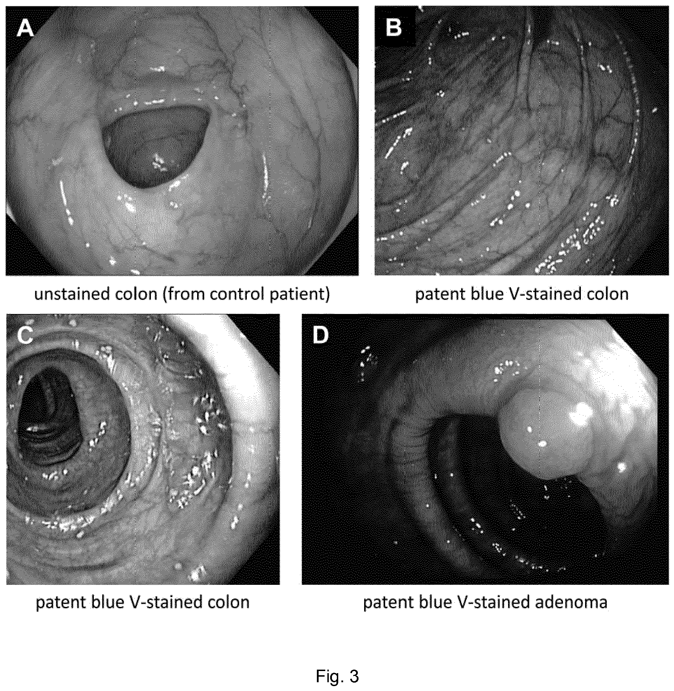

Figure 3 shows exemplary photographs comparing an unstained section of the ascending colon during conventional colonoscopy from a control patient (A), a patent blue V-stained section of the ascending colon after using the dye microcapsules of the present invention for chromocolonoscopy (B) as well as further staining examples (C, typical overview; D, stained adenoma).

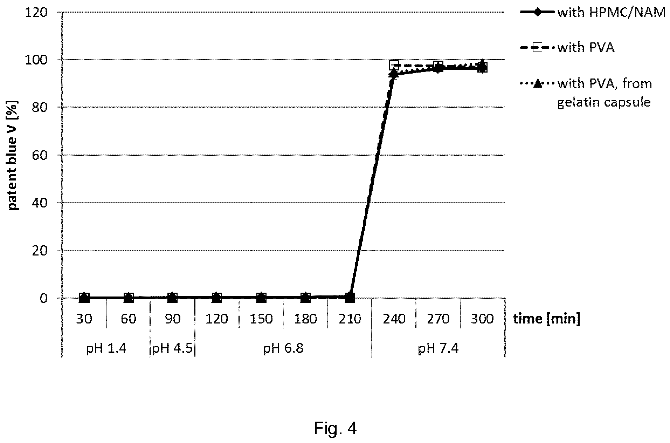

Figure 4 shows the pH-adapted *in vitro* release profiles of patent blue V from the microcapsule batch used for the first-in-man study containing the excipients HPMC and NAM (Example 1) and from the microcapsule batch produced with PVA as an excipient for granulation (Example 4) before and after encapsulation in gelatin hard capsules. The graphs represent means ± SD (n = 2). Percentage release refers to the total mass of patent blue V in the microcapsules.

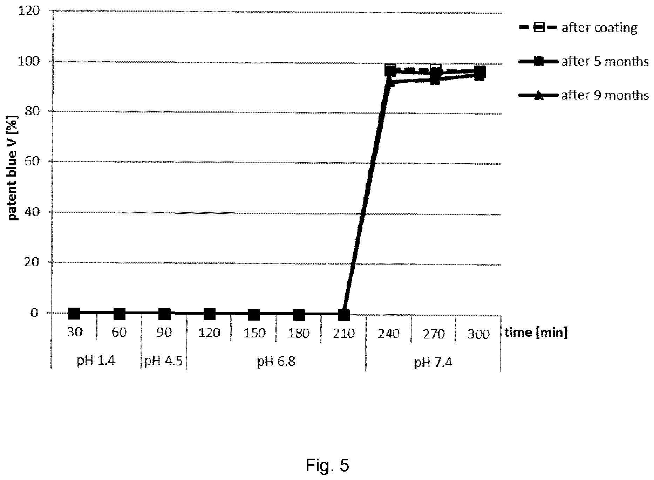

Figure 5 shows the pH-adapted *in vitro* release profiles of patent blue V from the microcapsule batch produced with PVA as an excipient for granulation (Example 4) after coating and after up to 9 months of storage at room temperature and protected from light. The graphs represent means ± SD (n = 2). Percentage release refers to the total mass of patent blue V in the microcapsules.

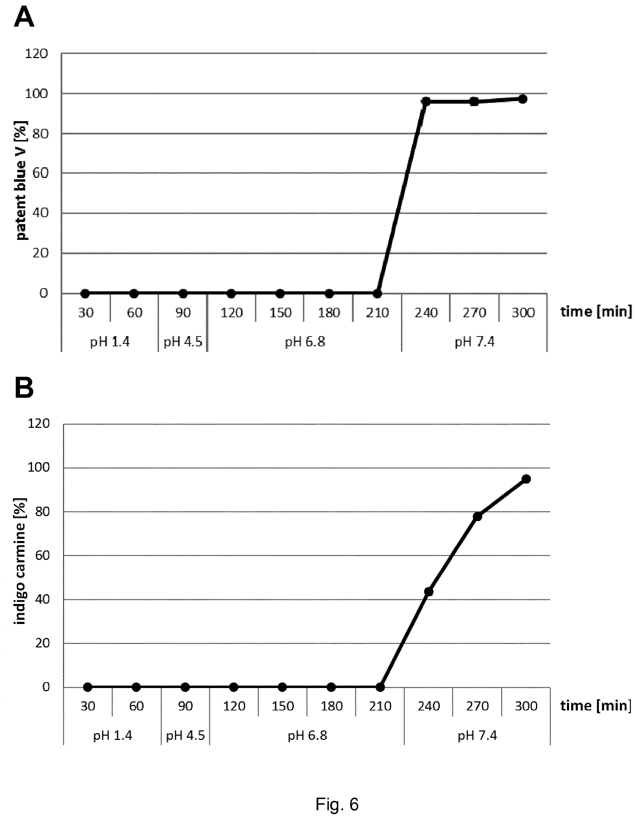

Figure 6 shows the pH-adapted *in vitro* release profiles of patent blue V (A) and indigo carmine (B) from microcapsule batches produced with PVA as an excipient for granulation (Example 5) after coating. The graphs represent means ± SD (n = 2). Percentage release refers to the total mass of dye in the microcapsules.

### DETAILED DESCRIPTION

The core of the present invention is a microcapsule comprising a core containing an active substance (1) and a coating layer system comprising an inner layer of shellac (2), an outer layer of shellac (3) and a pH-modulating substance (4) provided between the two layers of shellac (2, 3), with the proviso that the active substance is not vitamin B3.

Herein, the term "active substance" comprises any chemical, compound, combination, composition or mixture that has a direct effect on the gastrointestinal tract and/or an indirect effect on the body after resorption of the active substance by the gastrointestinal tract. In a preferred embodiment, the active substance comprises one or more dyes suitable for chromoendoscopy.

Herein, the term "vitamin B3" refers to nicotinic acid and/or nicotinamide, both alone or in combination, and the active substance is explicitly not vitamin B3. The exclusion of vitamin B3 as an active substance has only legal and no technical reasons, as a parallel application is filed describing microcapsules comprising vitamin B3 as the active substance.

Herein, the term "pH-modulating substance" comprises any chemical, compound, combination, composition, mixture or buffer system that may modulate the pH. In the present invention, the pH-modulating substance has the function to fine-tune and control the disintegration of the shellac coatings. The pH-modulating substance may be provided with or without excipients, e.g., as an intermediate layer between the two shellac coating layers. Combinations of two or more pH-modulating chemicals, also divergent in nature (e.g., acidic and/or basic substances with different pKa and/or pKb, combinations of weak and strong acids and/or bases) are also within the scope of the present invention. Therefore, the terms "basic substance" or "acidic substance" as used herein are not limiting in that such a substance should contain only basic or acidic components, respectively, but refer to the overall effect and properties of the pH-modulating substance. For example, such a substance may also comprise components which may be pH-neutral, basic (in the case of an overall acidic substance) or acidic (in the case of an overall basic substance).

Herein, the "lower small intestine" is the second half (length) of the small intestine.

Herein, the words "preferred" or "preferably" refer to embodiments that may have certain benefits under certain circumstances, but other embodiments may also be preferred under the same or other circumstances. The recitation of one or more preferred embodiments does not imply exclusion of other useful embodiments from the scope of the invention. Terms like "comprises" and variations thereof do not have a limiting meaning in the description and claims. Citation of certain sections of documents from the literature does not imply that the rest of such documents is not relevant or not incorporated by reference. The recitations of numerical ranges by one or two endpoints includes all numbers subsumed within that range (e.g., "1 to 10" includes 1, 2.4, 4.576, etc., and "lower than 1" includes all numbers smaller than 1). For any method disclosed or cited herein that includes discrete steps, the steps may be conducted in any feasible order, and any combination of two or more steps may be conducted simultaneously. Any example or list of examples should not be interpreted as a restriction of any kind or as an exclusive list.

In the case that the active substance-containing core of the microcapsule is overall acidic, the pH-modulating substance may be overall basic to partially counteract and control the stabilising acidic effect of the core on the shellac coatings in order to enable release of the active substance already at the pH prevalent, e.g., in the small intestine, with or without a prolonged release in the colon. In addition to the dual shellac layer, the use of a basic pH-modulating substance is also novel over the state of the art. Components of such basic pH-modulating substances are preferably selected from hydrogen carbonate, carbonate and/or acetate salts, e.g., sodium hydrogen carbonate (sodium bicarbonate), sodium carbonate, potassium hydrogen carbonate (potassium bicarbonate), potassium carbonate, ammonium hydrogen carbonate (ammonium bicarbonate), ammonium carbonate, calcium hydrogen carbonate (calcium bicarbonate), calcium carbonate, sodium acetate and/or calcium acetate.

Thus, one embodiment of the microcapsule according to the present invention is characterised in that the active substance-containing core is overall acidic, and the pH-modulating substance comprises or consists of a basic substance, preferably selected from the group consisting of hydrogen carbonate, carbonate salts, acetate salts, and their mixtures. In a preferred embodiment, the basic substance comprises or consists of sodium hydrogen carbonate (sodium bicarbonate).

In the case that the active substance-containing core of the microcapsule is overall neutral or basic, the pH-modulating substance may be overall acidic to subtly reduce the disintegration of the shellac coatings, resulting in prolonged shellac stability until reaching the desired part of the intestine, e.g., a burst release of the active substance in the small intestine, with or without a continued release in the colon. Components of such acidic pH-modulating substances are preferably selected from organic and/or inorganic acids, e.g., citric acid, malic acid, tartaric acid, ascorbic acid, sorbic acid, lactic acid, acetic acid and/or phosphoric acid.

Thus, one embodiment of the microcapsule according to the present invention is characterised in that the active substance-containing core is overall neutral or basic, and the pH-modulating substance comprises or consists of an acidic substance, preferably selected from the group consisting of organic acids, inorganic acids, and their mixtures. In a preferred embodiment, the acidic substance comprises or consists of citric acid.

The present invention also refers to a method for producing a microcapsule and/or a composition as described herein, comprising the steps of
a) providing a core material comprising or consisting of an active substance,
b) coating the core material with a first shellac layer,
c) providing a pH-modulating substance,
d) applying the pH-modulating substance onto the first shellac layer, e.g. in the form of an intermediate layer, and
e) applying a second shellac layer.

The core comprising or consisting of an active substance may be produced, e.g., by granulation of one or more active substances with or without excipients and/or other substances, or it may contain an excipient structure (e.g., a Cellet core) onto or into which the active substance(s) with or without excipients and/or other substances are applied, e.g., by spray coating. Furthermore, a core comprising or consisting of one or more active substances may also be coated with the same or other active substance(s).

For producing the core, the shellac layers and/or the pH-modulating substance, numerous excipients can be used, e.g., but not limited to, maltodextrin, glycerol, cellulose ethers [e.g., hydroxypropylmethylcellulose (HPMC), hydroxypropylcellulose (HPC), methylcellulose, ethylcellulose, and/or carboxymethylcellulose], polyvinyl alcohol (PVA, e.g., PVA 4-88), polyethylene oxide, carbopol polymers, polyacrylic acid, polysaccharides (e.g., xanthan gum, guar gum, chitosan, alginate, pectin, carrageenan, tragacanth, and/or different types of starch, e.g. pea starch and/or rice starch), polyvinylpyrrolidone and/or silicon dioxide. Two non-limiting examples for such procedures and the resulting microcapsules are provided in Example 1.

Depending on the size, structure and weight of the core, the amount of shellac and/or pH-modulating substance(s) applied in each step influences the percentage weight gain and the resulting size and properties of the microcapsule. The smaller the core and the more uneven its surface structure, regardless of its inherent composition and structure, the more surface it has in relationship to its volume, and the more shellac may have to be applied in relationship to the weight of the core in order to form an inner shellac layer with the desired properties. In addition, the shellac layers and the pH-modulating substances may be applied in different amounts, thicknesses and percentage weight gains, depending, e.g., on the intended release profile, the species of the subject which shall ingest the microcapsule (human or animal, see below), and/or the structure and composition of the core and the coating materials used (e.g., the particular properties and specifications of the shellac batch, pH-modulating substance and/or excipients). Herein, the percentage weight gain of each process step relates to the weight of the starting material used in this process step. For example, a weight gain of 10% by applying the outer shellac layer means that the microcapsules produced by this process step have 10% more weight than the starting material of this step, which already comprises the core, the inner shellac layer and the pH-modulating substance.

In the case that core structure of the microcapsule comprises or consists of one or more active substances, the percentage weight gain resulting from applying the inner shellac layer is 0.1-100%, preferably 0.25-90%, more preferably 0.5-80% and most preferably 2-60%. In the case that one or more active substances are sprayed onto a core structure, e.g., a Cellet core, the percentage weight gain resulting from applying the inner shellac layer is 0.1-100%, preferably 0.2-50%, more preferably 0.3-40% and most preferably 0.5-30%. The percentage weight gain of applying the pH-modulating substance is 0.1-30%, preferably 0.1-25% and most preferably 0.2-20%. Finally, the percentage weight gain resulting from applying the outer shellac layer is 0.1-100%, preferably 0.5-80%, more preferably 1-60% and most preferably 2-40%.

A preferred embodiment of the microcapsule according to the present invention is characterised in that the active substance comprises or consists of a dye suitable for chromoendoscopy. In a preferred embodiment, the active substance comprises or consists of a dye selected from the group consisting of allura red AC/FD&C red no. 40 (E129); alphazurine; alumina; amaranth (E123); anazolene sodium; annatto/bixin/norbixin (E160b); anthocyanins (E163); beetroot red/betanin (E162); β-apo-8'-carotenal (E160e); brilliant black BN/black PN (E151); brilliant blue FCF/FD&C blue no. 1 (E133); brown FK (E154); brown HT (E155); calcium carbonate (E170); canthaxanthin (E161g); capsanthian/capsorubin/paprika (E160c); carmoisine/azorubine (E122); carotenes (E160a); caramel (E150a-d); cochineal/carminic acid/carmines (E120); carotenes; carthamin; chlorophylls/chlorophyllins (E140); copper complexes of chlorophylls and/or chlorophyllins (E141); citrus red no. 2; cochineal red A/Ponceau 4R (E124); congo red; curcumin (E100); cresyl violet; D&C green no. 5; D&C yellow no. 10; diiodofluorescein; eosine/D&C red no. 22; erythrosine/FD&C red no. 3 (E127); Evans blue; fast green FCF (E143); FD&C green no. 3; flaming red/D&C red no. 36; fluorescein/uranine; green S (E142); helindone pink CN/D&C red no. 30; indanthrene blue (E130); indigotine/indigo carmine/FD&C blue no. 2 (E132); indocyanine green; iron oxides and hydroxides; isosulfan blue; lithol rubine B/D&C red no. 6; lithol rubine B Ca/D&C red no. 7; lithol rubine BK (E180); Lugol's solution; lutein (E161b); lycopene (E160d); methyl blue; methylene blue; orange B; patent blue V (E131); patent blue VF/sulfan blue; phenol red; phloxine B/D&C red no. 28; quinoline yellow (E104); riboflavin/riboflavin-5'-phosphate (E101); saffron (E164); spirulina extract; sudan black B; sunset yellow FCF/orange yellow S/FD&C yellow no. 6 (E110); tartrazine/FD&C yellow no. 5 (E102); tetrabromofluorescein/D&C red no. 21; tetrachlorotetrabromofluorescein/D&C red no. 27; titanium dioxide (E171); toluidine blue/tolonium chloride; turmeric (E100); vegetable carbon (E153); and derivatives, equivalents and mixtures thereof.

In a particularly preferred embodiment, the active substance comprises or consists of one or more dyes selected from the group consisting of patent blue V, isosulfan blue, indigo carmine, brilliant black BN, methylene blue, toluidine blue, cresyl violet and/or congo red.

In another preferred embodiment, the core of the microcapsule comprises or consists of combinations of dyes.

In yet another preferred embodiment, the core comprising or consisting of one dye (*e.g.*, brilliant black BN) may be coated with another dye (*e.g.*, patent blue V) with or without excipients before applying the inner shellac layer.

In yet another preferred embodiment, either the direct fluorescence (*e.g.*, methylene blue, patent blue V), the indirect fluorescence (*e.g.*, patent blue V, when bound to proteins) or the fluorescence-quenching properties (*e.g.*, brilliant black BN) of dyes from the group above may be favourably used to enhance the performance of endoscopy techniques employing a restricted light spectrum (*e.g.*, narrow band imaging endoscopy).

Depending on the core, the active substance(s) and their properties (*e.g.*, their pH), the composition of the pH-modulating substance and the construction of the dual shellac layers, the present invention also comprises use of the microcapsule as a medicament, medical product, diagnostic product, nutraceutical, dietary supplement, food ingredient or food. For example, formulations comprising the microcapsule may be used to deliver pharmacologically active substances, substances useful as dietary supplements, probiotics, prebiotics, synbiotics, dyes and/or other active substances to any part of the intestine, where they may act locally (*e.g.*, by staining the intestinal wall or by modifying the intestinal microbiota and/or their interaction with the intestines) and/or systemically (*e.g.*, due to absorption into the circulation and/or due to indirect systemic effects triggered by local effects on the intestine).

For clarification, the inventors use the following definitions:
Probiotic: ingestible live microbial cultures, which survive transit through the gastrointestinal tract and beneficially affect the host by improving its intestinal microbial balance. An example for a probiotic is VSL#3.

Prebiotic: non-digestible and selectively fermented food ingredient or supplement that allows specific changes in the composition and/or activity of the gastrointestinal microbiota which are beneficial for host well-being and health. Examples for prebiotics are resistant starch, fructo-oligosaccharides, galacto-oligosaccharides, xylooligosaccharides, polydextrose, lactulose, inulin or soluble fibre (*e.g.*, psyllium husk or acacia fibres).

Synbiotics: a combination of pro- and prebiotics.

In particular, the present invention relates to use of the microcapsule for chromoendoscopy of the small and/or large intestine for the diagnosis, therapy and/or prophylaxis of diseases and/or syndromes associated with and/or accompanied by intestinal inflammation, dysplasia, carcinogenesis and/or changes in the intestinal epithelium and/or intestinal mucosa and/or intestinal wall.

The use of the microcapsule for diagnostics is self-evident in this context, but this use indirectly also implies use in the prophylaxis (*e.g.*, by enabling the detection of intestinal precancerous or cancerous structures and/or lesions) and/or therapy (*e.g.*, by enabling their targeted removal) of such diseases and/or syndromes. Non-limiting examples for such diseases and/or syndromes are one or more selected from the group consisting of inflammatory and/or malignant diseases of the small intestine and/or colon; inflammatory bowel diseases, Crohn's disease, ulcerative colitis, indeterminate colitis; irritable bowel syndrome; diverticulitis; cancer of the small intestine; colorectal cancer; adenocarcinoma of the small intestine and/or colon; carcinoid tumor; lymphoma; gastrointestinal stromal tumor; sarcoma; leiomyosarcoma; melanoma; squamous cell carcinoma and other diseases and/or syndromes associated with and/or accompanied by intestinal inflammation, dysplasia, carcinogenesis and/or changes in the intestinal epithelium and/or intestinal mucosa and/or intestinal wall.

As used herein, the terms "therapy", "treatment" and "treat" refer to reversing, alleviating, delaying the onset of, or inhibiting the progress of a disease or disorder, or one or more symptoms thereof, as described herein. In some embodiments, such treatment may be administered after one or more symptoms have developed. In other embodiments, treatment may be administered in the absence of symptoms. For example, treatment may be administered to a susceptible individual prior to the onset of symptoms (e.g., in light of a history of symptoms and/or in light of genetic or other susceptibility factors). Treatment may also be continued after symptoms have resolved, for example to prevent or delay their recurrence, *e.g.* in remission maintenance of a chronic relapsing disorder.

As used herein, the terms "prophylaxis", "prevention" and "prevent" refer to delaying the onset of or reducing the likelihood of developing a disease or disorder or one or more symptoms thereof, as compared to an untreated control population.

Thus, the present invention also relates to use of the microcapsule for chromoendoscopy of the small and/or large intestine for the diagnosis, therapy and/or prophylaxis of diseases and/or syndromes selected from the group consisting of inflammatory and/or malignant diseases of the small intestine and/or colon; inflammatory bowel diseases, Crohn's disease, ulcerative colitis, indeterminate colitis; irritable bowel syndrome; diverticulitis; cancer of the small intestine; colorectal cancer; adenocarcinoma of the small intestine and/or colon; carcinoid tumor; lymphoma; gastrointestinal stromal tumor; sarcoma; leiomyosarcoma; melanoma; squamous cell carcinoma and other diseases and/or syndromes associated with and/or accompanied by intestinal inflammation, dysplasia, carcinogenesis and/or changes in the intestinal epithelium and/or intestinal mucosa and/or intestinal wall.

The claimed microcapsules are equally usable for the diagnostics and/or therapy and/or prophylaxis of diseases and/or syndromes with similar genesis in both human and other mammals, in particular in domestic and useful animals. Examples of such animals are dogs, cats, horses, camels, cattle or pigs without objective restriction.

In addition, the present invention also refers to a composition comprising a microcapsule of the invention. In a preferred embodiment, such a composition is formulated for oral administration with controlled and/or delayed release of the active substance(s) so that it releases (e.g., partially releases, selectively releases) in the small intestine and/or in the colon.

In the present invention, the terms "formulation" or "composition" or "treatment" or "prevention", and in particular the term "composition", have a broad meaning of a pharmaceutically and/or nutritionally and/or physiologically acceptable formulation, composition and/or mode of administration of the said active substance(s), which includes, but is not limited to, pharmaceutical formulations in the sense of medicaments (drugs), medical products, diagnostic products, nutraceuticals, dietary supplements, food ingredients and/or foods, also depending on the dose of the active substance(s) and the nature of the formulation. Preferred are medicaments, medical products, diagnostic products, nutraceuticals, and dietary supplements.

The term "controlled release" refers preferably to a pharmaceutical formulation or component thereof that releases, or delivers, one or more active ingredients over a prolonged period of time (time-dependent release) and/or under certain physiological conditions (e.g., pH-dependent release). In certain embodiments, the period of time or the release according to physiological conditions (e.g., pH) is sufficient for at least a portion of the active substances in a formulation to release in the small intestine (*e.g.*, in the lower small intestine) and/or in the colon.

The term "delayed release" relates preferably to a pharmaceutical formulation that releases the active ingredients after a period of delay. In certain embodiments, the delay is sufficient for at least a portion of the active substances in a formulation to release in the small intestine (*e.g.*, in the lower small intestine) and/or in the colon.

Depending on the nature and severity of the disease or condition as well as individual patient or subject characteristics, the dosage forms are administered once or several times daily, or in another dosage regimen to be chosen by a physician in the case of medicaments, medical products, diagnostic products or, in the case of nutraceuticals and/or dietary supplements, as defined by the instructions to the consumer.

The total dosage of one or more dyes suitable for chromoendoscopy administered in microcapsules and/or compositions according to the invention depends on the staining protocol, but will preferably be at or below the ADI for each dye according to current legislation.

As a non-limiting example, the European ADI of the preferred food colours patent blue V, indigo carmine and brilliant black BN is 5 mg/kg body weight for preparations with at least 90% purity (EFSA Panel 2010, EFSA J. 8:1540; EFSA Panel 2013, EFSA J. 11:2818; EFSA Panel 2014, EFSA J. 12:3768; see Background).

In a preferred embodiment, the use of the microcapsules and/or compositions of the present invention will accompany the bowel preparation for endoscopy, preferably colonoscopy, *e.g.*, according to the current guidelines of the European Society for Gastrointestinal Endoscopy (Hassan et al. 2013, Endoscopy 45:142) and/or the American Society for Gastrointestinal Endoscopy (ASGE Standards of Practice Committee 2015, Gastrointest. Endosc. 81:781).

As a non-limiting example, the bowel preparation for a colonoscopy according to the present invention may be performed by drinking 4 L of a bowel preparation solution in a split dose (*e.g.*, 2 L on the afternoon and evening of the day before colonoscopy and 2 L in the early morning of the day on which the colonoscopy is performed before the afternoon). The first dose of microcapsules and/or a composition containing such microcapsules may then, *e.g.*, be taken after the first 2 L in the evening, whereas a second dose after 3 L and a third dose after 4 L are taken the next morning. As further non-limiting examples, the dose of the bowel preparation solution may be split into 3 L on the day before and 1 L on the day of colonoscopy, or the whole bowel preparation solution may be taken on the day of colonoscopy (*e.g.*, if the colonoscopy is performed in the afternoon), or the total volume of bowel preparation solution may be lower (*e.g.*, 2 L) or higher (*e.g.*, 5 L). In addition, the timing and/or dosing regimen of the microcapsules and/or compositions may be freely and differentially adapted to the timing and/or dosing regimen of the bowel preparation solutions and vice versa.

Variations of such dosing regimens for bowel preparation solutions and/or chromoendoscopy dyes and/or microcapsules and/or formulations and/or compositions according to the invention may also depend, *e.g.*, on the brand and/or composition of such bowel preparation solutions, individual patient requirements, the intestinal region to be preferably stained, the nature of the dye(s) used, the kind of disease and/or syndrome afflicting the patient, the dose of the microcapsules contained in a formulation (unit) and many other factors which are evident to the person skilled in the art. However, the dosing regimens explicitly mentioned in this text may be preferred in some cases.

For oral administration, the microcapsules of the invention may, for example, be administered in free form (*e.g.*, mixed with food, beverages, probiotics, prebiotics or synbiotics), formulated in simple or coated tablets or dragees together with further excipients, or filled into capsules or sachets. The tablets are usually round or biconvex. Oblong tablet forms, which allow the tablet to be separated, are also possible.

The composition can also contain further excipient substances, such as binders, fillers, glidants, lubricants and/or flow regulating agents. The compositions according to the invention can be formulated, where appropriate, together with further active substances and with excipients conventional in pharmaceutical and/or nutritional compositions, *e.g.*, talcum, gum arabic, lactose, starch, magnesium stearate, cocoa butter, aqueous and non-aqueous carriers, lipid components of animal or vegetable origin, paraffin derivatives, glycols (in particular polyethylene glycol), various plasticizers, dispersants, emulsifiers, preservatives and/or other excipients as, *e.g.*, specified in the Examples below.

Accordingly, the present invention also relates to a composition for use for chromoendoscopy of the small and/or large intestine for the diagnosis, therapy and/or prophylaxis of diseases and/or syndromes selected from the group consisting of inflammatory and/or malignant diseases of the small intestine and/or colon; inflammatory bowel diseases, Crohn's disease, ulcerative colitis, indeterminate colitis; irritable bowel syndrome; diverticulitis; cancer of the small intestine; colorectal cancer; adenocarcinoma of the small intestine and/or colon; carcinoid tumor; lymphoma; gastrointestinal stromal tumor; sarcoma; leiomyosarcoma; melanoma; squamous cell carcinoma and other diseases and/or syndromes associated with and/or accompanied by intestinal inflammation, dysplasia, carcinogenesis and/or changes in the intestinal epithelium and/or intestinal mucosa and/or intestinal wall.

In another preferred embodiment, the present invention also comprises combination preparations of (a) one or more active substances selected from the same group of substances according to the invention (e.g., variable dose combinations or fixed dose combinations of two or more dyes), and (b) one or more active substances selected from one group according to the invention (e.g. dyes as described above) with one or more active substance(s) selected from another group of substances according to the invention (e.g., variable dose combinations or fixed dose combinations with probiotics, prebiotics or synbiotics to support the regeneration of the intestinal microbiota after the bowel preparation for endoscopy). The combinations described herein may be present in the same or separate dosage forms, which may be administered simultaneously, sequentially or on separate occasions.

While it is preferred according to the invention that the composition and/or the microcapsule according to the invention comprise both one or more active substances and one or more additional components, it may be beneficial to have separate compositions each comprising one or more of the active substances to be administered simultaneously or sequentially under a treatment or prevention regimen. Of note, such a set of compositions is per definition for this application a composition according to the invention, too.

As used herein, the term "variable dose combination" refers to a combination of two or more active substances in medicaments, medical products, diagnostic products, nutraceuticals or dietary supplements, whereby each of these substances is applied in the form of a separate composition, e.g., two single dosage forms. The separate compositions may be administered simultaneously, sequentially or on separate occasions by an administration regimen. In a preferred embodiment, microcapsules of the invention containing one or more active substances may be combined in variable dose combinations and may be administered simultaneously, sequentially or on separate occasions with separate compositions. These variable dose combinations may use conventionally available compositions or may be also achieved, *e.g.*, by customised polypharmacy or compounding.

In contrast to a variable dose combination, a "fixed-dose combination" is defined as a combination medicament, medical product, diagnostic product, nutraceutical or dietary supplement which is a formulation including two or more active ingredients, *e.g.*, active substances, combined in a single dosage form, which is manufactured and distributed in certain respective fixed doses. A fixed-dose combination mostly refers to a mass-produced product having a predetermined combination of active substances and respective dosages (as opposed to, e.g., customised polypharmacy or compounding). For example, microcapsules of the invention containing one or more active substances may be combined in fixed dose combinations by including a specific ratio of these microcapsules in a larger dosage form, *e.g.*, a capsule, tablet or sachet.

Thus, the microcapsules of the present invention can be formulated to comprise combinations of active substances, but the invention also encompasses combinations of such microcapsules with other active substances and/or compositions. For example, the components may also be physically segregated even within the same dosage form, e.g. by adding microcapsules to a probiotic, a prebiotic, a synbiotic or any other substance or composition, or by filling microcapsules according to the invention and one or more controlled or delayed release formulations, *e.g.* microcapsules or granules, of other active substances into one capsule for easier administration. Thus, the invention also pertains to a composition, which is a controlled and/or delayed release formulation of an active substance alone, or a variable dose combination or, preferably, a fixed dose combination of a controlled and/or delayed release formulation of an active substance with a probiotic, a prebiotic, a synbiotic or any other substance, such substance being preferably formulated for controlled and/or delayed release.

Therefore, the present invention also relates to microcapsules and compositions comprising variable and/or fixed dose combinations with one or more other active substances and/or compositions.

A further aspect of the invention described herein is the efficient use of the claimed medicaments, medical products, diagnostic products, nutraceuticals, dietary supplements, food ingredients or foods on the basis of genetic and/or microbiological and/or blood parameter and/or other biomarker data and specific needs of the individuals to be treated. For example, new insights into the genetic predisposition of individuals for all types of diseases and into pharmacogenetics indicate that an evidence-based personalised medicine including genetic analyses of relevant risk genes and also of genes which code *e.g.*, for cell surface receptors, transporter proteins, metabolism enzymes or signal transduction proteins, which interact with an active substance and/or its metabolites and/or its downstream effectors, can contribute information and improvements with respect to the type of use, the mode of application, the time(s) of use, the dose and/or the dosage regimen of the medicaments, medical products, diagnostic products, nutraceuticals, dietary supplements, food ingredients or foods described herein. Individuals who may benefit from this personalised treatment include those with disease-specific or non-specific changes in blood biomarkers and/or other biomarkers. This applies analogously to analyses of the intestinal microbiota, particularly when a stool sample indicates a change in the microbiota. The present invention thus also comprises the use of suitable genetic and/or microbiological and/or blood parameter and/or other biomarker test methods to identify individuals particularly susceptible to or benefiting from the medicaments, medical products, diagnostic products, nutraceuticals, dietary supplements, food ingredients or foods according to the invention and/or to adapt their use according to the invention to the individual circumstances. This also comprises expressly the use of the active substances or their combinations with other active substances in different modes of administration depending on the genetic and microbiological properties of the individual. For these purposes, it is possible to use laboratory tests and/or suitable test kits and also measuring methods, devices and/or kits to be employed by a physician, user and/or patient, *e.g.*, to take stool samples or to analyse suitable parameters in the blood, urine or other body fluids. In particular, the present invention also relates to using the intestinal microbiota in part and/or in their entirety (the microbiome) as biomarkers, to support patient or subject selection for the treatments or preventions described herein, to personalise and adapt the compositions and/or treatments and/or preventions described herein, and/or to determine end points and efficacy benchmarks for the compositions and/or treatments and/or preventions described herein.

### EXEMPLIFICATION

There are variable possibilities to advantageously develop, and develop further, the teaching of the present invention. For this purpose, reference is made to the examples below which describe the invention in a representative way.

If not indicated otherwise, the meaning of "%" is "% by weight".

### Example 1: Production and characterisation of dye microcapsules

One embodiment of the present invention is that cores comprising the food colours patent blue V and/or brilliant black BN as active substances are coated by two layers of shellac, which are separated by an intermediate layer of a pH-modulating substance (Figure 1). The use of patent blue V and/or brilliant black BN in the present example served a dual purpose. From a mechanistic point of view, the visible release of these dyes in the targeted parts of the intestine was the proof of principle of the release any active substance according to the broader scope of the invention. At the same time, the example was also required to illustrate the successful use of dye-containing microcapsules for chromoendoscopy of the colon according to one particular embodiment of the invention. In the microcapsules used in this example, nicotinamide was used as a food-grade excipient with stable pH properties (pH 6.61) and previously proven good granulation properties in this system, not as an active substance. Citric acid was used as an acidic pH-modulating substance to control shellac dissociation of the dye-containing microcapsules.

### Equipment and materials

**Table 1: Equipment used for the preparation and characterisation of the microcapsules**

| | |
|---|---|
| Capsule filler | Aponorm® capsule filler for 60 capsules with size 0 plate set; WEPA Apothekenbedarf, Hillscheid, Germany |
| Dissolution tester | Model DT 70; Pharmatest Group, Hainburg, Germany |
| Fluidized bed coater | Mini Glatt; Glatt Ingenieurtechnik, Binzen, Germany |
| Granulator | ProCell Labsystem with ProCell 005 spouted bed insert; Glatt Ingenieurtechnik, Binzen, Germany |
| Quartz cuvette | SUPRASIL®; Type-No. 100-QS; 10 mm; Heraeus, Hanau, Germany |
| Spectrophotometer | Helios Gamma, UVG145021; Thermo Fisher Scientific, Dreieich, Germany |

**Table 2: Materials used for the preparation of the microcapsules and capsules**

| | |
|---|---|
| Brilliant black BN | Brilliant black BN (E151); Fiorio Colori SpA, Gessate (MI), Italy |
| Citric acid monohydrate | Citric acid monohydrate for food use, test sample; Jungbunzlauer, Basel, Switzerland |
| Gelatin capsules | Coni-Snap® capsules, yellow, size 0, batch no. 34037611; Capsugel, Morristown, NJ, USA |
| Hydroxypropylmethylcellulose (HPMC) | Pharmacoat 606; Shin-Etsu Chemical Co., Tokyo, Japan |
| Maltodextrin | C* Dry maltodextrin 01915, batch no. 02023411; Cargill, Minneapolis, MN, USA |
| Nicotinamide (NAM) | Nicotinamide, 10V2002; batch no. 169256; SternVitamin, Ahrensburg, Germany |
| Patent blue V | Patent blue V Ca (E131); Fiorio Colori SpA, Gessate (MI), Italy |
| Shellac | 0560 0003, SSB® Aquagold, 25% shellac ammonium salt, batch no. 168920; Stroever, Bremen, Germany |

**Table 3: Materials used for the preparation of the dissolution buffers**

| | | |
|---|---|---|
| Citric acid anhydrous | X863.2, >99.5%, batch no. 452193486 | Carl Roth, Karlsruhe, Germany |
| Di-sodium hydrogen phosphate dihydrate | 4984.1, >99.5%, batch no. 175226790 | |
| Hydrochloric acid | 4625.1, 37%, batch no. 433206404 | |
| Potassium dihydrogen phosphate | 3904.1, >99%, batch no. 234214969 | |
| Sodium chloride | 3957.2, >99.5%, batch no. 483205341 | |
| Tri-sodium citrate dihydrate | 3580.3, >99%, batch no. 123186150 | |

### Methods

### Preparation of coating solutions

The composition of each formulation part is listed in Table 4. The ingredients were dissolved at room temperature under moderate stirring in tap water (H₂O).

**Table 4: Composition of the granules and coatings**

| **Coating** | **Ingredient 1** | % | **Ingredient 2** | % | **Ingredient 3** | % | **H₂O** % |
|---|---|---|---|---|---|---|---|
| **Granulation** | Dye (patent blue V or brilliant black BN) | 15 | Nicotinamide | 15 | HPMC | 1.2 | 68.8 |
| **pH coating** | Citric acid | 1 | Maltodextrin | 9 | - | - | 90 |
| **Inner and outer shellac coatings** | Shellac SSB® Aquagold (25%) | 60 | - | - | - | - | 40 |

### Production of dye microcapsules

For producing the granule cores, spray solutions containing 15% dye (patent blue V or brilliant black BN) and 15% nicotinamide plus 1.2% pharmacoat 606 (HPMC; 4% based on dry weight) in water were prepared (Table 4), 150 g of maltodextrin DE 15 were provided as starting material, and a continuous granulation process was performed in a ProCell granulator with a ProCell 005 spouted bed insert.

Compared to granules containing only the excipient nicotinamide (data not shown), the dye granules were more sticky and asymmetrically shaped. The average size of the resulting granules was 358 µm. In the next step, the inner shellac coating was applied to 100 g of the dye granules (diameter 315-400 µm) in a Mini Glatt fluid bed coater with bottom spray (Glatt, Binzen, Germany) using a 0.5 mm two-way nozzle and an atomizing air pressure of 0.55-0.6 bar. Inlet air pressure was adjusted to 0.35-0.5 bar, and the inlet air temperature was set to 40°C, which resulted in a product temperature of about 33.8°C. The spraying rate was increased from 0.36 to 0.64 g/min. The final weight gain was about 46.5% (patent blue V granules) or 48.0% (brilliant black BN granules). After this first shellac coating step, the microcapsules were dried at 50°C in a drying oven for 1 h, and agglomerates with a diameter of more than 500 µm were removed by sieving. The citric acid intermediate coating was applied under the following conditions to 130 g of the shellac-coated dye granules: atomizing air pressure of 0.53-0.65 bar, inlet air pressure of 0.41-0.55 bar, inlet air temperature of 41-42°C, product temperature of about 36.3°C and an increasing spraying rate from 0.36 to 0.52 g/min. The calculated weight gain was 1% (referred to citric acid) and 10% (referred to total dry mass including maltodextrin). The outer shellac coating was applied under the same conditions as the inner shellac coating except for the inlet air pressure (0.52-0.58 bar) and atomizing air pressure (0.62-0.66 bar), which were higher because of the weight gain. The calculated weight gain was 10% for the outer shellac coating (referring to the weight of the microcapsules after the first shellac coating step), and the measured combined weight gain for the citric acid and outer shellac layers was a total of 18.85% (1.15% loss compared to the added calculated weight gains of 20%). After the second shellac coating step, the microcapsules were again dried at 50°C in a drying oven for 1 h, and agglomerates with a diameter of more than 500 µm were removed by sieving. After first tests of this procedure showing insufficient gastric resistance, the outer shellac layer was expanded in further experiments with additional 10% calculated and 9.3% measured weight gain for microcapsules with patent blue V or with additional 5% calculated and 3.9% measured weight gain for microcapsules with brilliant black BN.

### In vitro dissolution testing

Dissolution tests were performed at 37°C with 0.5 g of dye microcapsules in 250 ml simulated gastric fluid (pH 1.4; Table 5), citrate buffer (pH 4.5; Table 5) or phosphate buffers (pH 6.8 or 7.4; Table 5) using a standard paddle apparatus at 100 rpm. Dissolution experiments were run for 1 h at pH 1.4, 0.5 h at pH 4.5, 2 h at pH 6.8 and 1.5 h at pH 7.4. Every 30 min, dye release was recorded spectrophotometrically using Quartz cuvettes at 416 nm (patent blue V in pH 1.4), 637 nm (patent blue V in other buffers) or 571 nm (brilliant black BN). Before each measurement, samples were diluted (patent blue V: 1:50; brilliant black BN: 1:12.5).

**Table 5: Composition of buffers for the in vitro dissolution tests**

| **Buffer** | **pH** | **Ingredient 1** | **Amount or %** | **Ingredient 2** | **Amount or %** | **Solvent** | **Vol.** |
|---|---|---|---|---|---|---|---|
| **Simulated gastric fluid** | 1.4 | Sodium chloride | 2g | Hydrochloric acid (6 M) | 7 ml | dH₂O* | ad 1 L |
| **Citrate buffer** | 4.5 | Citric acid monohydrate | 4.8 g | Tri-sodium-citrate dihydrate | 7.35 g | dH₂O* | ad 1 L |
| **Phosphate buffer** | 6.8 | Potassium dihydrogen phosphate (0.91%)* | 50.8% | Di-sodium hydrogen phosphate dihydrate (1.19%)* | 49.2% | - | - |
| **Phosphate buffer** | 7.4 | Potassium dihydrogen phosphate (0.91%)* | 18.2% | Di-sodium hydrogen phosphate dihydrate (1.19%)* | 81.8% | - | - |

| | | | | | | | |
|---|---|---|---|---|---|---|---|
| * Solvent: demineralised water (dH₂O). | | | | | | | |

### Determination of the total content of patent blue V or brilliant black BN in coated microcapsules

In order to calculate the percentage of dye release, the total content of patent blue V or brilliant black BN in the microcapsules was determined spectrophotometrically at 637 nm (patent blue V) or 571 nm (brilliant black BN) in triplicate with 0.5 g microcapsules in 250 ml phosphate buffer (pH 7.4) in a standard paddle apparatus at 37°C and 100 rpm. After 30, 60 and 90 minutes, samples were taken, diluted (patent blue V: 1:50; brilliant black BN: 1:12.5) and measured.

### Capsule filling

The microcapsules containing patent blue V or brilliant black BN were filled into standard size 0 gelatin capsules using a manual capsule filler for 60 capsules. A maximum of approximately 0.53 g of microcapsules fit into one gelatin capsule. In the FIM study, differential dosing was achieved by administration of differential amounts of capsules.

### Results and discussion

A comparison between the release profiles obtained for patent blue V (Figure 2A) and brilliant black BN (Figure 2B) illustrates how the release profiles of the microcapsules of the invention can be modulated by rather small differential weight gains (only approximately 5% more shellac on patent blue V microcapsules), and how the dual shellac layer with an intermediate coating with a pH-modulating substance can provide diverse desired pH-dependent release profiles normally not achieved with shellac, which solves an important problem of shellac coatings reported in the state of art (Limmatvapirat et al. 2007, Eur. J. Pharm. Biopharm. 67:690; Czarnocka & Alhnan 2015, Int. J. Pharm. 486:167). The total dye contents of the microcapsules as determined spectrophotometrically were 184.6 ± 0.43 mg of patent blue V per gram of microcapsules (mean ± SD; n = 3) and 206.6 ± 0.87 mg brilliant black BN/g microcapsules (mean ± SD; n = 3), respectively. In order to ensure comparability of exposure and results during the first-in-man (FIM) study (see Example 2), the stability of the release profiles of the microcapsules was monitored. As shown in Figure 2, the release profiles of the patent blue V microcapsules did not change significantly over time up to 12 months of storage. For the brilliant black BN microcapsules, only 6 months of storage were investigated and also did not show any significant deterioration. Taken together, the analysis of the characteristics and stability of the dye microcapsules clearly supported their suitability for controlled release of patent blue V or brilliant black BN in the FIM study.

### Example 2: First-in-man (FIM) study

### Aims of the study

In the FIM study, size 0 gelatin capsules filled with well-characterised batches of microcapsules containing patent blue V or brilliant black BN (see Example 1) were administered to outpatients undergoing endoscopy of the gastrointestinal tract. The aims of the study were (1) to investigate whether the *in vitro* release profiles of the dye microcapsules translated into appropriate *in vivo* release (general proof of concept for the microcapsules of the present invention) and (2) to analyse the suitability of the dye microcapsules for chromoendoscopy of the colon (special proof of concept for representative food-grade chromoendoscopy dyes as active substances).

For study aim (2), the primary objective was to demonstrate sufficient staining of the complete colon, and the secondary objective was to indirectly compare possible side effects with the published rates of methylene blue MMX® tablets (Repici et al. 2012, Contemp. Clin. Trials. 33:260). The primary endpoint was scoring of intestinal epithelial staining with TSC/NSA scores according to PCT/EP2013/070060 (example 5) leading to a mean staining score (TSC, see definition in the Methods section) of at least 10 and at least a mean of two stained areas (of the four areas ascending colon, transverse colon, descending colon and sigma/rectum) with a staining score of ≥2 (cf. administration schedule J for 200 mg in the cited example). The secondary endpoint was a rate of adverse events as defined by Repici et al. (Contemp. Clin. Trials. 33:260, 2012) in <40% of patients.

### Methods

### Study population and design

The study was performed at the Interdisciplinary Endoscopy Unit of the Department of Internal Medicine 1 of the University Hospital Schleswig-Holstein, Campus Kiel (Kiel, Germany). The study was approved by the ethics committee of the University of Kiel (reference number: D439/15) and registered at ClinicalTrials.gov with the study number NCT02631798. Written informed consent was obtained from each patient.

As summarised in Table 6, eight patient volunteers scheduled for screening colonoscopy were included and allocated to the following groups receiving two dose levels of microencapsulated patent blue V (E131) and/or one dose level of microencapsulated brilliant black BN (E151) (see Example 1).

**Table 6: Baseline characteristics and doses of the FIM study population**

| **No.** | **Gender** | **Age** | **Dose (gelatin capsules)¹** | **Total daily dose (mg)²** |
|---|---|---|---|---|
| **1** | male | 45 | (1+1)+(2) E131 / (0+0)+(0) E151 | 189 E131 / 0 E151 |
| **2** | male | 76 | (1+1)+(2) E131 / (0+0)+(0) E151 | 189 E131 / 0 E151 |
| **3³** | male | 44 | (0+0)+(0) E131 / (1+1)+(2) E151 | 0 E131 / 219 E151 |
| **4** | female | 63 | (0+0)+(0) E131 / (1+1)+(2) E151 | 0 E131 / 219 E151 |
| **5** | male | 33 | (2+2)+(4) E131 / (0+0)+(0) E151 | 378 E131 / 0 E151 |
| **6** | male | 41 | (2+2)+(4) E131 / (0+0)+(0) E151 | 378 E131 / 0 E151 |
| **7³** | male | 46 | (1+1)+(2) E131 / (1+1)+(2) E151 | 189 E131 / 219 E151 |
| **8** | female | 48 | (1+1)+(2) E131 / (1+1)+(2) E151 | 189 E131 / 219 E151 |
| ¹ The number of capsules in brackets refer to day 1 (after 2 L and after 3 L of bowel preparation solution) and day 2 (after 4 L), respectively (see section "Treatment and dosing regimen" below); | | | | |
| ² each E131 gelatin capsule contained approximately 94.5 mg of E131, each E151 gelatin capsule contained approximately 109.5 mg of E151; | | | | |
| ³ Patients 3 and 7 had to be excluded due to deviations from protocol: patient 3 changed the bowel preparation solution after 1 L, and patient 7 drank much more clear fluid than requested until the stool was colourless. | | | | |

### Treatment and dosing regimen:

Due to the upper systemic intake limit of 5 mg/kg for both patent blue V (E131) and brilliant black BN (E151) (EFSA Panel 2010, EFSA J. 8:1540; EFSA Panel 2013, EFSA J. 11:2818) and the low systemic exposure expected both from the *in vitro* release profiles targeting the ileum and from human data with similar controlled release microcapsules (not shown), a maximum dose of approximately 400 mg per dye and day was chosen. The E131 and E151 microcapsules were administered in size 0 gelatin hard capsules (Conisnap, Capsugel) in order to facilitate dosing (Example 1). The microcapsules had the following dye contents: E131: 184.6 mg/g ± 0.43 (mean ± standard deviation; n = 3); E151: 206.6 mg/g ± 0.87 (mean ± standard deviation; n = 3) (Example 1). With 0.51-0.53 g of microcapsules per capsule, each capsule contained approximately 100 mg of dye (see below). The following batches of microcapsules were produced at the Institute of Food Technology of the University of Kiel from 05 to 08 October 2015 and filled into capsules on 02 December 2015:
E131-02Dec15-1: 0.512 g of microcapsules per capsule, *i.e.*, approximately 94.5 mg E131 per capsule; 60 capsules;
E151-02Dec15-1: 0.530 g of microcapsules per capsule, *i.e.*, approximately 109.5 mg E151 per capsule; 112 capsules.

The staining efficacy of E131 and E151 when released from the novel microcapsules of the invention was unknown and investigated for the first time in the present study. Therefore, the planned sample size was not derived from a statistical power calculation. The test administration schedules were based on procedures and results from different studies and publications (Repici et al. 2012, Contemp. Clin. Trials. 33:260; Danese et al. 2013, 8th Congress of ECCO, Poster Presentations: Clinical: Diagnosis and Outcome, P194; US8545811; PCT/EP2013/070060).

The laxative Klean-Prep (Norgine, Marburg, Germany) was administered following the Klean-Prep manual for patients at the Interdisciplinary Endoscopy Unit of the Department of Internal Medicine 1 of the University Hospital Schleswig-Holstein, Campus Kiel (Kiel, Germany). Patients drank 3 L of Klean-Prep within 3 h (ideally, 250 mL every 15 min) starting at 17:00 h on the day before colonoscopy. From 06:00 h to 07:00 h on the day of colonoscopy, patients drank the last 1 L of Klean-Prep. Deviations from protocol as well as observations of effects and side effects were documented in a patient diary.

In order to allow sufficient staining, colonoscopy was not performed before at least 5 hours had passed since the ingestion of the last capsules (i.e., when the capsules were taken according to plan at 07:00 h, colonoscopy started at or after 12:00 h).

When 4 capsules were administered, patients took 1 capsule after the second quarter of Klean-Prep (after 2 L; at 19:00 h), 1 capsule after the third quarter (after 3 L; at 20:00 h) and 2 capsules at the end of the bowel preparation (after 4 L; at 07:00 h).

When 8 capsules were administered, patients took 2 capsules after the second quarter of Klean-Prep (after 2 L; at 19:00 h), 2 capsules after the third quarter (after 3 L; at 20:00 h) and 4 capsules at the end of the bowel preparation on the next morning (after 4 L; at 07:00 h).

### Inclusion criteria

- Healthy males and post-menopausal females aged 30 to 80 years and scheduled for screening colonoscopy and optionally endoscopy of the upper gastrointestinal tract;
- body weight ≥60 kg;
- good health based on medical history, physical examination, a 12-lead electrocardiogram (ECG) and routine haematology and blood chemistry tests;
- ability to understand and comply with the protocol;
- written informed consent.

### Exclusion criteria

- Standard criteria for bioavailability estimation of new drugs, namely (i) intake of any medication, (ii) a history of drug, caffeine (>5 cups coffee/tea/day) or tobacco (≥10 cigarettes/day) abuse, (iii) history of alcohol consumption in excess of two drinks per day in males and one drink per day in females;
- known or suspected hypersensitivity to food colourants, especially E131 or E151;
- gastrointestinal obstruction or perforation;
- serious cardiovascular, renal or hepatic disease;
- prolonged prothrombin time, elevated INR (international normalised ratio);
- elevated serum creatinine;
- any other severe underlying medical condition.

### Analysis and scoring:

In order to determine the intake of capsules, a patient diary was completed by the study subjects. In addition to the standard assessments of screening colonoscopy, the staining efficacy was scored with a system derived from example 5 of PCT/EP2013/070060 with a number between 0 and 20, calculated as a sum of individual staining scores for each colonic section investigated (ascending colon, transverse colon, descending colon and sigma/rectum) ranging from 0 to 5 (0, "unstained"; 1, "traces", i.e., weak dye traces; 2, "detectable", 25-50% of the area appropriately stained; 3, "acceptable", 50-75% of the area appropriately stained; 4, "good", 75-100% of the area appropriately stained; 5, "overstated", overstaining not enabling the endoscopist to see the mucosal surface with the due accuracy in 100% of the area). Accordingly, the optimal result was 16 with a score of 4 in all four areas of the colon.

In addition, the endoscopy personnel was asked to judge the performance of the dye capsules according to the following criteria with a simple scoring system (+, good/yes; o, average/undecided; -, bad/no; n/a, not applicable): "general staining intensity", "contrast of lesions", "contrast of polyps", "contrast of flat adenoma", "better than spraying technique" and "cleanliness of equipment".

### Results and discussion

Six of eight patients completed the study according to protocol (Table 6). The results summarised in Table 7 confirm that the microcapsules solved the problems defined in the Background section:
(1) The study met the primary endpoint with a mean colon staining score >10 (actually: 13.3) and a mean of two stained colonic segments (NSA) with a staining score >2 (actually: 3.7). The mucosal staining efficacy was on average 3.3 (of 4), with the best staining (4 of 4) in the ascending colon, in which lesion detection is most difficult according to the prior art.
(2) No adverse events were observed. Therefore, the study also met the secondary endpoint with the rate of adverse events as defined by Repici et al. (Contemp. Clin. Trials. 33:260, 2012) in <40% of patients.
(3) In 4 of 5 patients in which both the upper and lower gastrointestinal tract were inspected, the microcapsules selectively and exclusively released - as planned - in the lower small intestine (ileum) and colon. The staining of the stomach in Patient 4 indicates mechanical damage to some microcapsules during the encapsulation into gelatin capsules, which could be reproduced in control *in vitro* experiments with retained samples (data not shown).
(4) In 4 of 6 patients, the staining result was better than that usually observed with conventional spraying techniques, and in the remaining 2 of 6 patients, it resembled the treatment standard. In Patient 8, a standard spraying chromoendoscopy had been performed four days earlier, allowing a direct comparison between the two procedures and a clear superiority of the microcapsule staining procedure, as reported by the endoscopist.
(5) In both patients with flat adenoma, the adenoma was well highlighted by the staining according to the present invention.
(6) No overstaining, which could potentially mask lesions or other diagnostically important areas, was observed.
(7) The duration of procedure was the same as with conventional colonoscopies. In contrast, chromoendoscopy using a spraying catheter needs approximately twice the time (additional 20 min).
(8) The cleanliness of equipment was better than with conventional spraying chromoendoscopy.

**Table 7: Results of the FIM study**

| **Criteria** | **P1** | **P2** | **P4** | **P5** | **P6** | **P8** |
|---|---|---|---|---|---|---|
| Total duration of colonoscopy (min) | 20 | 13 | 21 | 18 | 17 | 13 |
| Staining score ¹ of esophagus | 0 | 0 | 0 | n/a² | 0 | 0 |
| Staining score of stomach | 0 | 0 | 3 | n/a | 0 | 0 |
| Staining score of duodenum | 0 | 0 | 0 | n/a | 0 | 0 |
| Staining score of ileum | 4 | 4 | 4 | n/a | 4 | 4 |
| Staining score of caecum | 4 | n/a | 4 | 4 | 4 | 4 |
| Staining score of ascending colon³ | 4 | 4 | 4 | 4 | 4 | 4 |
| Staining score of transverse colon³ | 4 | 4 | 3 | 3 | 3 | 3 |
| Staining score of descending colon³ | 4 | 4 | 2 | 2 | 4 | 3 |
| Staining score of sigma/rectum³ | 4 | 4 | 1 | 1 | 4 | 3 |
| Score⁴ for "general staining intensity" | + | + | ○ | ○ | + | + |
| Score for "contrast of lesions" | n/a | n/a | n/a | n/a | n/a | n/a |
| Score for "contrast of polyps" | n/a | n/a | n/a | n/a | n/a | n/a |
| Score for "contrast of flat adenoma" | n/a | + | n/a | n/a | n/a | + |
| Score for "better than spraying technique" | + | + | ○ | ○ | + | + |
| Score for "cleanliness of equipment" | + | + | + | + | + | + |
| ¹ Staining score system as defined in "Methods": 0, "unstained"; 1, "traces"; 2, "detectable", 25-50% of the area appropriately stained; 3, "acceptable", 50-75% of the area appropriately stained; 4, "good", 75-100% of the area appropriately stained; 5, "overstained"; | | | | | | |
| ² n/a, not applicable (stained area: not inspected/inspectable; scores: not found); | | | | | | |
| ³ the four colonic areas used to calculate the mean colon staining score; | | | | | | |
| ⁴ Score for further criteria as defined in "Methods": +, good/yes; ○, average/undecided; -, bad/no; n/a, not applicable. | | | | | | |

Figure 3 shows an exemplary comparison between an unstained section of the ascending colon during conventional colonoscopy and a stained section of the ascending colon after using the dye microcapsules of the present invention for chromocolonoscopy.

In summary, these results support both the general concept of targeted release from the microcapsules of the invention as well as the particular concept of the superior usefulness of such microcapsules for chromoendoscopy compared to the current clinical practice.

### Example 3: Production of microcapsules with methylene blue and indigo carmine

The techniques described in Example 1 are advantageously used to produce further types of microcapsules comprising other dyes preferred according to the invention, namely methylene blue and indigo carmine. In contrast to the contrast dye indigo carmine, which pools in grooves and crevices after release from the shellac microcapsules, methylene blue is absorbed by the intestinal epithelium, with the advantage that the staining with methylene blue microcapsules is less likely to be compromised by excessive fluid intake.

### Example 4: Production and characterisation of patent blue V microcapsules with the granulation excipient polyvinyl alcohol

In order to further optimise the granulation process, polyvinyl alcohol (PVA) was tested as an alternative excipient.

### Methods

### Production of dye microcapsules

Patent blue V microcapsules with PVA cores were prepared with a process similar to the one for HPMC/NAM cores described in Example 1, but with the following modifications:
(1) the batch no. of patent blue V was FI23497;
(2) PVA (Mowiol 4-88; article no. 0713; Carl Roth, Karlsruhe, Germany) was used as an excipient for granulation instead of the combination of HPMC and nicotinamide;
(3) the spray solution contained 9.5% patent blue V plus 0.95% PVA (10% PVA based on the dry weight of patent blue V) in water (89.55%);
(4) patent blue granules from another experiment with 25% HPMC were used as starting material;
(5) the granulator for the continuous granulation process featured a Vario 3 spouted bed insert.

The average size of the resulting granulated cores was between 315 and 400 µm.

In the next step, the inner shellac coating was applied to 100 g of the patent blue V granules in a Mini Glatt fluid bed coater with bottom spray (Glatt, Binzen, Germany) using a 0.5 mm two-way nozzle and an atomizing air pressure of 0.6 bar. Inlet air pressure was adjusted to 0.35-0.45 bar, and the inlet air temperature was set to 40°C, which resulted in a product temperature of about 33.8°C. The spraying rate was increased from 0.36 to 0.64 g/min. The weight gain was 50.0%. After this first shellac coating step, the microcapsules were dried at 50°C in a drying oven for 1 h, and particles with a diameter of less than 250 µm were removed by sieving. No agglomerates were observed. The citric acid intermediate coating was applied under the following conditions to 95 g of the shellac-coated patent blue V granules: atomizing air pressure of 0.4 bar, inlet air pressure of 0.55 bar, inlet air temperature of 42-44°C, product temperature of about 36.0°C and an increasing spraying rate from 0.38 to 0.56 g/min. The calculated weight gain was 1% (referred to citric acid) and 10% (referred to total dry mass including maltodextrin). The outer shellac coating was applied under the same conditions as the inner shellac coating. The calculated weight gain was 18% for the outer shellac coating, and the measured combined weight gain for the citric acid and outer shellac layer was a total of 26.32% (1.68% loss compared to the added calculated weight gains of 28%). After the second shellac coating step, the microcapsules were again dried at 50°C in a drying oven for 1 h.

### In vitro dissolution testing and determination of the total content of patent blue V

Dissolution tests were performed as described in Example 1 with one exception: before each measurement, samples were diluted 1:100.

### Results and discussion

The patent blue V cores obtained in the PVA granulation process showed a roundness comparable with that of the HPMC/NAM cores described in Example 1. In a control experiment, HPMC without NAM was used for granulation but produced suboptimal results compared to PVA due to higher solution viscosity (data not shown). In comparison to HPMC and NAM as excipients as described in Example 1, the patent blue V content of the microcapsules was improved with PVA. The total patent blue V content of the microcapsules as determined spectrophotometrically was 399.4 ± 2.86 mg of patent blue V per gram of microcapsules with PVA as granulation binder compared to 184.6 ± 0.43 mg in the microcapsules from Example 1 with HPMC as granulation binder (mean ± SD; n = 3).

The weight gains in the coating steps compared to the respective intermediate product were as follows:
HPMC/NAM microcapsules: 100 plus 46.5% weight gain (inner coating) plus 18.85% weight gain (intermediate and outer coating) plus 9.3% weight gain (expanded outer coating), resulting in a total coating weight gain of 90.3% (46.5% plus 27.6% plus 16.2% referring to the patent blue V cores set as 100%).

PVA microcapsules: 100 plus 50.0% weight gain (inner coating) plus 26.32% weight gain (intermediate and outer coating), resulting in a total coating weight gain of 89.5% (50.0% plus 39.5% referring to the patent blue V cores set as 100%).

Thus, the overall weight gains due to coating were practically identical for the HPMC (90.3%) and PVA (89.5%), with differences in individual steps compensated across the complete production process.

The theoretical proportion of patent blue V in the cores of the two types of microcapsules was calculated as follows:
HPMC/NAM microcapsule cores: 15% patent blue V, 15% nicotinamide, 1.2% pharmacoat 606 (HPMC); proportion of patent blue V of dry weight: 15 / 31.2 = 48.1%.

PVA microcapsule cores: 9.5% patent blue V, 0.95% PVA; proportion of patent blue V of dry weight: 9.5 / 10.45 = 90.9%.

Therefore, the theoretical increase in patent blue V content of the PVA microcapsules compared to the HPMC microcapsules - given approximately the same weight gain due to the coating steps - was expected to be no more than approximately 1.89-fold (90.9%/48.1%).

Surprisingly, however, the patent blue V content of the PVA microcapsules was approximately 2.16-fold higher than that of the HPMC microcapsules (399.4 mg/g vs. 184.6 mg/g, see above). This unexpected 14% increase in patent blue V content (2.16-fold instead of 1.89-fold) is advantageous for the microcapsules of the present invention, as a required amount of patent blue V can be delivered in a smaller amount of microcapsules. This feature has the potential to both lower the cost of goods and increase patient comfort and compliance by allowing smaller packaging capsules (e.g. gelatin capsules), which are easier to swallow.

A comparison between the release profiles obtained for the HPMC/NAM/patent blue V microcapsules from Example 1 and the alternative PVA/patent blue V microcapsules demonstrated almost identical release properties with a >90% burst release under conditions of the terminal ileum (Figure 4).

Interestingly, whereas a minor fraction of microcapsules with HPMC/NAM cores (Example 1) were mechanically damaged during encapsulation in gelatin hard capsules (Example 2), no such damage was observed with the microcapsules with PVA-containing cores (an exemplary release profile of a test batch after removal from filled gelatin capsules is shown in Figure 4).

Finally, Figure 5 shows that the release profile of the PVA/patent blue V microcapsules did not change during up to 9 months of storage.

### Example 5: Production and characterisation of patent blue V and indigo blue microcapsules with the granulation excipient polyvinyl alcohol (PVA)

In order to compare release profiles of patent blue V and indigo carmine from cores granulated with the excipient PVA (Example 4), new microcapsule batches were coated and tested back to back.

### Methods

### Production of dye microcapsules

Patent blue V cores were the same as described in Example 4. Indigo carmine cores were granulated as described in Example 4 using a spray solution with 20% dry mass consisting of 90% indigo carmine and 10% PVA. The granule size varied between 315 and 400 µm. The three coating layers were applied in the same fashion as described in Example 4.

### In vitro dissolution testing and determination of the total content of patent blue V and indigo carmine

Dissolution tests were performed as described in Example 1 with the following exceptions: before each measurement, patent blue V samples were diluted 1:100, and indigo carmine samples were diluted 1:25 and measured at a wavelength of 610 nm.

### Results and discussion

The indigo carmine cores obtained in the granulation process showed a roundness comparable with the patent blue V cores described in Example 4. The release profile of indigo carmine microcapsules was slightly delayed at pH 7.4 compared with the release profile of the patent blue V microcapsules (Figure 6). The total dye contents of the microcapsules determined spectrophotometrically were 379.39 ± 0.87 mg patent blue V per gram microcapsules and 465.29 ± 2.42 mg indigo carmine per gram microcapsules (mean ± SD; n = 3).

## Claims

1. A microcapsule comprising a core containing an active substance (1) and a coating layer system comprising an inner layer of shellac (2), an outer layer of shellac (3) and a pH-modulating substance (4) provided between the two layers of shellac (2, 3), with the proviso that the active substance is not vitamin B3.

2. The microcapsule according to claim 1, **characterised in that** the active substance-containing core is overall acidic, and the pH-modulating substance comprises or consists of a basic substance, preferably selected from the group consisting of hydrogen carbonate, carbonate salts, acetate salts, and their mixtures.

3. The microcapsule according to claim 1, **characterised in that** the active substance-containing core is overall neutral or basic, and the pH-modulating substance comprises or consists of an acidic substance, preferably selected from the group consisting of organic acids, inorganic acids, and their mixtures.

4. The microcapsule according to claim 1 or 2, **characterised in that** the pH-modulating substance comprises or consists of sodium hydrogen carbonate (sodium bicarbonate).

5. The microcapsule according to claim 1 or 3, **characterised in that** the pH-modulating substance comprises or consists of citric acid.

6. The microcapsule according to any one of claims 1 to 5, **characterised in that** the active substance comprises or consists of a dye suitable for chromoendoscopy.

7. The microcapsule according to any one of claims 1 to 6, **characterised in that** the active substance comprises or consists of a dye selected from the group consisting of allura red AC/FD&C red no. 40 (E129); alphazurine; alumina; amaranth (E123); anazolene sodium; annatto/bixin/norbixin (E160b); anthocyanins (E163); beetroot red/betanin (E162); β-apo-8'-carotenal (E160e); brilliant black BN/black PN (E151); brilliant blue FCF/FD&C blue no. 1 (E133); brown FK (E154); brown HT (E155); calcium carbonate (E170); canthaxanthin (E161g); capsanthian/capsorubin/paprika (E160c); carmoisine/azorubine (E122); carotenes (E160a); caramel (E150a-d); cochineal/carminic acid/carmines (E120); carotenes; carthamin; chlorophylls/chlorophyllins (E140); copper complexes of chlorophylls and/or chlorophyllins (E141); citrus red no. 2; cochineal red A/Ponceau 4R (E124); congo red; curcumin (E100); cresyl violet; D&C green no. 5; D&C yellow no. 10; diiodofluorescein; eosine/D&C red no. 22; erythrosine/FD&C red no. 3 (E127); Evans blue; fast green FCF (E143); FD&C green no. 3; flaming red/D&C red no. 36; fluorescein/uranine; green S (E142); helindone pink CN/D&C red no. 30; indanthrene blue (E130); indigotine/indigo carmine/FD&C blue no. 2 (E132); indocyanine green; iron oxides and hydroxides; isosulfan blue; lithol rubine B/D&C red no. 6; lithol rubine B Ca/D&C red no. 7; lithol rubine BK (E180); Lugol's solution; lutein (E161b); lycopene (E160d); methyl blue; methylene blue; orange B; patent blue V (E131); patent blue VF/sulfan blue; phenol red; phloxine B/D&C red no. 28; quinoline yellow (E104); riboflavin/riboflavin-5'-phosphate (E101); saffron (E164); spirulina extract; sudan black B; sunset yellow FCF/orange yellow S/FD&C yellow no. 6 (E110); tartrazine/FD&C yellow no. 5 (E102); tetrabromofluorescein/D&C red no. 21; tetrachlorotetrabromofluorescein/D&C red no. 27; titanium dioxide (E171); toluidine blue/tolonium chloride; turmeric (E100); vegetable carbon (E153); and derivatives, equivalents and mixtures thereof.

8. The microcapsule according to any one of claims 1 to 7, for use as a medicament, medical product, diagnostic product, nutraceutical, dietary supplement, food ingredient or food.

9. The microcapsule according to any one of claims 1 to 7 or the microcapsule for use according to claim 8, for use for chromoendoscopy of the small and/or large intestine for the diagnosis, therapy and/or prophylaxis of diseases and/or syndromes associated with and/or accompanied by intestinal inflammation, dysplasia, carcinogenesis and/or changes in the intestinal epithelium and/or intestinal mucosa and/or intestinal wall.

10. The microcapsule according to any one of claims 1 to 7 or the microcapsule for use according to any one of claims 8 to 9, for use for chromoendoscopy of the small and/or large intestine for the diagnosis, therapy and/or prophylaxis of diseases and/or syndromes selected from the group consisting of inflammatory and/or malignant diseases of the small intestine and/or colon; inflammatory bowel diseases, Crohn's disease, ulcerative colitis, indeterminate colitis; irritable bowel syndrome; diverticulitis; cancer of the small intestine; colorectal cancer; adenocarcinoma of the small intestine and/or colon; carcinoid tumor; lymphoma; gastrointestinal stromal tumor; sarcoma; leiomyosarcoma; melanoma; squamous cell carcinoma and other diseases and/or syndromes associated with and/or accompanied by intestinal inflammation, dysplasia, carcinogenesis and/or changes in the intestinal epithelium and/or intestinal mucosa and/or intestinal wall.

11. A composition comprising a microcapsule according to any one of claims 1 to 10.

12. The composition according to claim 11, formulated for oral administration with controlled and/or delayed release of the active substance.

13. The composition according to claims 11 or 12, for use for chromoendoscopy of the small and/or large intestine for the diagnosis, therapy and/or prophylaxis of diseases and/or syndromes selected from the group consisting of inflammatory and/or malignant diseases of the small intestine and/or colon; inflammatory bowel diseases, Crohn's disease, ulcerative colitis, indeterminate colitis; irritable bowel syndrome; diverticulitis; cancer of the small intestine; colorectal cancer; adenocarcinoma of the small intestine and/or colon; carcinoid tumor; lymphoma; gastrointestinal stromal tumor; sarcoma; leiomyosarcoma; melanoma; squamous cell carcinoma and other diseases and/or syndromes associated with and/or accompanied by intestinal inflammation, dysplasia, carcinogenesis and/or changes in the intestinal epithelium and/or intestinal mucosa and/or intestinal wall.

14. The microcapsule according to any one of claims 1 to 7 or the microcapsule for use according to any one of claims 8 to 10, or the composition according to any one of claims 11 to 12 or the composition for use according to claim 13, comprising variable and/or fixed dose combinations with one or more other active substances and/or compositions.

15. A method for producing a microcapsule according to any one of claims 1 to 7 or a microcapsule for use according to any one of claims 8 to 10 or a composition according to any one of claims 11 to 12 or a composition for use according to claim 13, comprising the steps of
a. providing a core material comprising or consisting of an active substance,
b. coating the core material with a first shellac layer.
c. providing a pH-modulating substance,
d. applying the pH-modulating substance onto the first shellac layer, and
e. applying a second shellac layer.

## Patentansprüche

1. Mikrokapsel, umfassend einen Kern, der eine aktive Substanz (1) enthält, und ein Beschichtungsschichtsystem, umfassend eine innere Schicht aus Schellack (2), eine äußere Schicht aus Schellack (3) und eine pH-modulierende Substanz (4), die zwischen den zwei Schichten von Schellack (2, 3) vorgesehen ist, mit der Maßgabe, dass der Wirkstoff nicht Vitamin B3 ist.

2. Mikrokapsel nach Anspruch 1, **dadurch gekennzeichnet, dass** der den Wirkstoff enthaltende Kern insgesamt sauer ist und die pH-modulierende Substanz eine basische Substanz umfasst oder daraus besteht, vorzugsweise ausgewählt aus der Gruppe, die aus Hydrogencarbonat, Carbonatsalzen, Acetatsalzen, und deren Mischungen besteht.

3. Mikrokapsel nach Anspruch 1, **dadurch gekennzeichnet, dass** der den Wirkstoff enthaltende Kern insgesamt neutral oder basisch ist und die pH-modulierende Substanz eine saure Substanz umfasst oder daraus besteht, vorzugsweise ausgewählt aus der Gruppe, die aus organischen Säuren, anorganischen Säuren und deren Mischungen besteht.

4. Mikrokapsel nach Anspruch 1 oder 2, **dadurch gekennzeichnet, dass** die pH-modulierende Substanz Natriumhydrogencarbonat (Natriumbicarbonat) umfasst oder daraus besteht.

5. Mikrokapsel nach Anspruch 1 oder 3, **dadurch gekennzeichnet, dass** die pH-modulierende Substanz Zitronensäure umfasst oder daraus besteht.

6. Mikrokapsel nach einem der Ansprüche 1 bis 5, **dadurch gekennzeichnet, dass** der Wirkstoff einen für die Chromoendoskopie geeigneten Farbstoff umfasst oder daraus besteht.

7. Mikrokapsel nach einem der Ansprüche 1 bis 6, **dadurch gekennzeichnet, dass** der Wirkstoff einen Farbstoff umfasst oder aus diesem besteht, ausgewählt aus der Gruppe bestehend aus Allurarot AC/FD&C Rot Nr. 40 (E129); Alphazurin; Aluminiumoxid; Amaranth (E123); Anazolennatrium; Annatto/Bixin/Norbixin (E160b); Anthocyane (E163); Rote Beete-Rot/Betanin (E162); β-apo-8'-carotinal (E160e); Brilliantschwarz BN / schwarzes PN (E151); Brillantblau FCF/FD&C Blau Nr. 1 (E133); Braun FK (E154); Braun HT (E155); Calciumcarbonat (E170); Canthaxanthin (E161g); Capsanthian / Capsorubin / Paprika (E160c); Carmoisin / Azorubin (E122); Carotin (E160a); Karamell (E150a-d); Cochineal / Carminsäure / Carmine (E120); Carotine; Carthamin; Chlorophylle / Chlorophylline (E140); Kupferkomplexen von Chlorophyllen und / oder Chlorophyllinen (E141); Zitrusrot Nr. 2; Cochinealrot A / Ponceau 4R (E124); Kongorot; Curcumin (E100); Kresylviolett; D&C Grün Nr. 5; D&C Gelb Nr. 10; Diiodofluorescein; Eosin / D&C-Rot Nr. 22; Erythrosin / FD&C-Rot Nr. 3 (E127); Evans Blau; Echtgrün FCF (E143); FD&C Grün Nr. 3; Flammendem Rot/ D&C Rot Nr. 36; Fluorescein / Uranin; Grün S (E142); Helindonrosa CN/D&C Rot Nr. 30; Indanthrenblau (E130); Indigotin / Indigokarmin / FD&C Blau Nr. 2 (E132); Indocyaningrün; Eisenoxide und -hydroxide; Isosulfanblau; Lithol Rubin B/D&C Rot Nr. 6; Lithol Rubin B Ca/D&C Rot Nr. 7; Litholrubin BK (E180); Lugols Lösung; Lutein (E161b); Lycopin (E160d); Methylblau; Methylenblau; Orange B; Patentblau V (E131); Patentblau VF / Sulfanblau; Phenolrot; Phloxin B/D&C Rot Nr. 28; Chinolingelb (E104); Riboflavin / Riboflavin-5'-phosphat (E101); Safran (E164); Spirulina-Extrakt; Sudanschwarz B; Sonnenuntergangs-Gelb FCF / Orange-Gelb S/FD&C Gelb Nr. 6 (E110); Tartrazin / FD&C Gelb Nr. 5 (E102); Tetrabromfluorescein / D&C Rot Nr. 21; Tetrachlortetrabromfluorescein / D&C Rot Nr. 27; Titandioxid (E171); Toluidinblau / Toloniumchlorid; Kurkuma (E100); pflanzlichem Kohlenstoff (E153); und Derivaten, Äquivalenten und Gemischen dieser.

8. Mikrokapsel nach einem der Ansprüche 1 bis 7 zur Anwendung als Medikament, medizinisches Produkt, diagnostisches Produkt, Nutrazeutikum, Nahrungsergänzungsmittel, Lebensmittelinhaltsstoff oder Lebensmittel.

9. Mikrokapsel nach einem der Ansprüche 1 bis 7 oder Mikrokapsel zur Anwendung nach Anspruch 8, zur Anwendung für die Chromoendoskopie des Dünn- und/oder Dickdarms für die Diagnose, Therapie und/oder Prophylaxe von Krankheiten und/oder Syndromen, die mit Darmentzündungen, Dysplasie, Karzinogenese und/oder Veränderungen des Darmepithels und/oder der Darmschleimhaut und/oder der Darmwand verbunden sind und/oder von diesen begleitet werden.

10. Mikrokapsel nach einem der Ansprüche 1 bis 7 oder Mikrokapsel zur Anwendung nach einem der Ansprüche 8 bis 9, zur Anwendung für die Chromoendoskopie des Dünn- und/oder Dickdarms für die Diagnose, Therapie und/oder Prophylaxe von Krankheiten und/oder Syndromen, ausgewählt aus der Gruppe bestehend aus entzündlichen und/oder bösartigen Erkrankungen des Dünndarms und/oder des Dickdarms; entzündlichen Darmerkrankungen, Morbus Crohn, Colitis ulcerosa, unbestimmter Colitis; Reizdarmsyndrom; Divertikulitis; Dünndarmkrebs; Darmkrebs; Adenokarzinom des Dünndarms und/oder Dickdarms; Karzinoid-Tumor; Lymphom; gastrointestinalem Stromatumor; Sarkom; Leiomyosarkom; Melanom; Plattenepithelkarzinom und anderen Krankheiten und/oder Syndromen, die mit Darmentzündungen, Dysplasie, Karzinogenese und/oder Veränderungen in dem Darmepithel und/oder der Darmschleimhaut und/oder der Darmwand verbunden sind und/oder von diesen begleitet werden.

11. Zusammensetzung, umfassend eine Mikrokapsel nach einem der Ansprüche 1 bis 10.

12. Zusammensetzung nach Anspruch 11, formuliert zur oralen Verabreichung mit kontrollierter und/oder verzögerter Freisetzung des Wirkstoffs.

13. Zusammensetzung nach den Ansprüchen 11 oder 12 zur Anwendung für die Chromoendoskopie des Dünn- und/oder Dickdarms für die Diagnose, Therapie und/oder Prophylaxe von Krankheiten und/oder Syndromen, ausgewählt aus der Gruppe bestehend aus entzündlichen und/oder bösartigen Erkrankungen des Dünndarms und/oder Dickdarms; entzündlichen Darmerkrankungen, Morbus Crohn, Colitis ulcerosa, unbestimmter Colitis; Reizdarmsyndrom; Divertikulitis; Dünndarmkrebs; Darmkrebs; Adenokarzinom des Dünndarms und/oder Dickdarms; Karzinoid-Tumor; Lymphom; gastrointestinalem Stromatumor; Sarkom; Leiomyosarkom; Melanom; Plattenepithelkarzinom und anderen Krankheiten und/oder Syndromen, die mit Darmentzündungen, Dysplasie, Karzinogenese und/oder Veränderungen in dem Darmepithel und/oder der Darmschleimhaut und/oder der Darmwand verbunden sind und/oder von diesen begleitet werden.

14. Mikrokapsel nach einem der Ansprüche 1 bis 7 oder Mikrokapsel zur Anwendung nach einem der Ansprüche 8 bis 10 oder Zusammensetzung nach einem der Ansprüche 11 bis 12 oder Zusammensetzung zur Anwendung nach Anspruch 13, umfassend Kombinationen von variabler und/oder fester Dosis mit einem oder mehreren anderen Wirkstoffen und/oder Zusammensetzungen.

15. Verfahren zum Herstellen einer Mikrokapsel nach einem der Ansprüche 1 bis 7 oder Mikrokapsel zur Anwendung nach einem der Ansprüche 8 bis 10 oder Zusammensetzung nach einem der Ansprüche 11 bis 12 oder Zusammensetzung zur Anwendung nach Anspruch 13, umfassend die Schritte
a. Bereitstellens eines Kernmaterials, das einen Wirkstoff umfasst oder daraus besteht,
b. Beschichten des Kernmaterials mit einer ersten Schellackschicht,
c. Bereitstellen einer pH-modulierenden Substanz,
d. Aufbringen der pH-modulierenden Substanz auf die erste Schellackschicht, und
e. Aufbringen einer zweiten Schellackschicht.

## Revendications

1. Microcapsule comprenant un noyau contenant une substance active (1) et un système de couches d'enrobage comprenant une couche interne de gomme laque (2), une couche externe de gomme laque (3) et une substance modulant le pH (4) fournie entre les deux couches de gomme laque (2, 3), à condition que la substance active ne soit pas la vitamine B3.

2. Microcapsule selon la revendication 1, **caractérisée en ce que** le noyau contenant une substance active est globalement acide, et la substance modulant le pH comprend ou consiste en une substance basique, de préférence sélectionnée dans le groupe consistant en un hydrogénocarbonate, les sels de carbonate, les sels d'acétate, et leurs mélanges.

3. Microcapsule selon la revendication 1, **caractérisée en ce que** le noyau contenant une substance active est globalement neutre ou basique, et la substance modulant le pH comprend ou consiste en une substance acide, de préférence sélectionnée dans le groupe consistant en les acides organiques, les acides inorganiques, et leurs mélanges.

4. Microcapsule selon la revendication 1 ou 2, **caractérisée en ce que** la substance modulant le pH comprend ou consiste en l'hydrogénocarbonate de sodium (bicarbonate de sodium).

5. Microcapsule selon la revendication 1 ou 3, **caractérisée en ce que** la substance modulant le pH comprend ou consiste en l'acide citrique.

6. Microcapsule selon l'une quelconque des revendications 1 à 5, **caractérisée en ce que** la substance active comprend ou consiste en un colorant approprié pour une chromoendoscopie.

7. Microcapsule selon l'une quelconque des revendications 1 à 6, **caractérisée en ce que** la substance active comprend ou consiste en un colorant sélectionné dans le groupe consistant en le rouge allura AC/FD&C rouge no. 40 (E129) ; l'alphazurine ; l'alumine ; l'amarante (E123) ; l'anazolène sodique ; le rocou/ bixine/norbixine (E160b) ; les anthocyanines (E163) ; le rouge de betterave/bétanine (E162) ; le β-apo-8'-caroténal (E160e) ; le noir brillant BN/noir PN (E151) ; le bleu brillant FCF/FD&C bleu no. 1 (E133) ; le brun FK (E154) ; le brun HT (E155) ; le carbonate de calcium (E170) ; la canthaxanthine (E161g) ; la capsanthine/capsorubine/paprika (E160c) ; la carmoisine/azorubine (E122) ; les carotènes (E160a) ; le caramel (E150a-d) ; la cochenille/acide carminique/ carmins (E120) ; les carotènes ; la carthamine ; les chlorophylles/chlorophyllines (E140) ; les complexes de cuivre de chlorophylles et/ou de chlorophyllines (E141) ; le rouge citrin no. 2 ; le rouge cochenille A/Ponceau 4R (E124) ; le rouge Congo ; la curcumine (E100) ; le violet de crésyle ; le D&C vert no. 5 ; le D&C jaune no. 10 ; la diiodofluorescéine ; l'éosine/D&C rouge no. 22 ; l'érythrosine/FD&C rouge no. 3 (E127) ; le bleu Evans ; le vert solide FCF (E143) ; le FD&C vert no. 3 ; le rouge flamboyant/D&C rouge no. 36 ; la fluorescéine/uranine ; le vert S (E142) ; le rose hélindone CN/D&C rouge no. 30 ; le bleu d'indanthrène (E130) ; l'indigotine/carmin d'indigo/FD&C bleu no. 2 (E132) ; le vert d'indocyanine ; les oxydes et hydroxydes de fer ; le bleu isosulfan ; le lithol rubine B/D&C rouge no. 6 ; le lithol rubine B Ca/D&C rouge no. 7 ; le lithol rubine BK (E180) ; la solution de Lugol ; la lutéine (E161b) ; le lycopène (E160d) ; le bleu de méthyle ; le bleu de méthylène ; l'orange B ; le bleu patenté V (E131) ; le bleu patenté VF/bleu sulfan ; le rouge de phénol ; la phloxine B/D&C rouge no. 28 ; le jaune de quinoléine (E104) ; la riboflavine/riboflavine-5'-phosphate (E101) ; le safran (E164) ; un extrait de spiruline ; le noir Soudan B ; le jaune soleil FCF/jaune orangé S/FD&C jaune no. 6 (E110) ; la tartrazine/FD&C jaune no. 5 (E102) ; la tétrabromofluorescéine/D&C rouge no. 21 ; la tétrachlorotétrabromofluorescéine/D&C rouge no. 27 ; le dioxyde de titane (E171) ; le bleu de toluidine/chlorure de tolonium ; le curcuma (E100) ; le carbone végétal (E153) ; et leurs dérivés, équivalents et mélanges.

8. Microcapsule selon l'une quelconque des revendications 1 à 7, pour une utilisation comme médicament, produit médical, produit de diagnostic, nutraceutique, complément alimentaire, ingrédient alimentaire ou aliment.

9. Microcapsule selon l'une quelconque des revendications 1 à 7 ou microcapsule pour une utilisation selon la revendication 8 pour une utilisation pour une chromoendoscopie de l'intestin grêle et/ou du gros intestin pour le diagnostic, la thérapie et/ou la prophylaxie de maladies et/ou de syndromes associés à et/ou accompagnés d'une inflammation intestinale, d'une dysplasie, d'une carcinogenèse et/ou de modifications de l'épithélium intestinal et/ou de la muqueuse intestinale et/ou de la paroi intestinale.

10. Microcapsule selon l'une quelconque des revendications 1 à 7 ou microcapsule pour une utilisation selon l'une quelconque des revendications 8 à 9, pour une utilisation pour une chromoendoscopie de l'intestin grêle et/ou du gros intestin pour le diagnostic, la thérapie et/ou la prophylaxie de maladies et/ou de syndromes sélectionnés dans le groupe consistant en les maladies inflammatoires et/ou malignes de l'intestin grêle et/ou du côlon ; les maladies inflammatoires chroniques de l'intestin, la maladie de Crohn, la rectocolite hémorragique, une colite indéterminée ; le syndrome du côlon irritable ; la diverticulite ; un cancer de l'intestin grêle ; un cancer colorectal ; un adénocarcinome de l'intestin grêle et/ou du côlon ; une tumeur carcinoïde ; un lymphome ; une tumeur stromale gastro-intestinale ; un sarcome ; un léiomyosarcome ; un mélanome ; un carcinome épidermoïde et d'autres maladies et/ou syndromes associés à et/ou accompagnés d'une inflammation intestinale, d'une dysplasie, d'une carcinogenèse et/ou de modifications de l'épithélium intestinal et/ou de la muqueuse intestinale et/ou de la paroi intestinale.

11. Composition comprenant une microcapsule selon l'une quelconque des revendications 1 à 10.

12. Composition selon la revendication 11, formulée pour une administration orale avec libération contrôlée et/ou retardée de la substance active.

13. Composition selon les revendications 11 ou 12, pour une utilisation pour une chromoendoscopie de l'intestin grêle et/ou du gros intestin pour le diagnostic, la thérapie et/ou la prophylaxie de maladies et/ou de syndromes sélectionnés dans le groupe consistant en les maladies inflammatoires et/ou malignes de l'intestin grêle et/ou du côlon ; les maladies inflammatoires chroniques de l'intestin, la maladie de Crohn, la rectocolite hémorragique, une colite indéterminée ; le syndrome du côlon irritable ; la diverticulite ; un cancer de l'intestin grêle ; un cancer colorectal ; un adénocarcinome de l'intestin grêle et/ou du côlon ; une tumeur carcinoïde ; un lymphome ; une tumeur stromale gastro-intestinale ; un sarcome ; un léiomyosarcome ; un mélanome ; un carcinome épidermoïde et d'autres maladies et/ou syndromes associés à et/ou accompagnés d'une inflammation intestinale, d'une dysplasie, d'une carcinogenèse et/ou de modifications de l'épithélium intestinal et/ou de la muqueuse intestinale et/ou de la paroi intestinale.

14. Microcapsule selon l'une quelconque des revendications 1 à 7 ou microcapsule pour une utilisation selon l'une quelconque des revendications 8 à 10, ou composition selon l'une quelconque des revendications 11 à 12 ou composition pour une utilisation selon la revendication 13, comprenant des combinaisons à dose variable et/ou fixe avec une ou plusieurs autres substances actives et/ou compositions.

15. Procédé de production d'une microcapsule selon l'une quelconque des revendications 1 à 7 ou d'une microcapsule pour une utilisation selon l'une quelconque des revendications 8 à 10 ou d'une composition selon l'une quelconque des revendications 11 à 12 ou d'une composition pour une utilisation selon la revendication 13, comprenant les étapes suivantes
a. la fourniture d'un matériau de noyau comprenant ou consistant en une substance active,
b. l'enrobage du matériau de noyau avec une première couche de gomme laque,
c. la fourniture d'une substance modulant le pH,
d. l'application de la substance modulant le pH sur la première couche de gomme laque, et
e. l'application d'une seconde couche de gomme laque.
